# EUROPEAN PATENT APPLICATION

(11) **EP 4 144 843 A1**
(43) Date of publication of application: **08.03.2023**
(21) Application number: 21796821.3
(22) Date of filing: 30.04.2021
(51) Int. Cl.: C12N 13/00, C12N 5/0781, C12N 5/0783, C12N 5/0784, C12N 5/0786, C12N 5/0787, A61K 35/17, A61K 35/15, A61P 35/00, A61P 35/02

(54) **IMMUNE CELL TREATED BY MEANS OF MAGNETIC FIELD AND USE THEREOF**

(30) Priority: 30.04.2020 CN 202010366867
(71) Applicant: Heye Health Technology Co., Ltd., Huzhou City, Zhejiang Province 313300 (CN); Center for Excellence in Molecular Cell Science, Chinese Academy of Sciences, Shanghai 200031 (CN)
(72) Inventor: ZHU, Xiaoyan, Shanghai 200031 (CN); FANG, Zhicai, HUZHOU CITY, Zhejiang 313300 (CN); FANG, Yanwen, HUZHOU CITY, Zhejiang 313300 (CN); LIU, Yan, Shanghai 200031 (CN); CAO, Xianxia, Shanghai 200031 (CN); LIU, Haifeng, Shanghai 200031 (CN)
(74) Representative: WSL Patentanwälte Partnerschaft mbB
(86) International application number: PCT/CN2021/091341
(87) International publication number: WO 2021/219119

(57) **Abstract**

Provided are an immune cell treated by means of a magnetic field and the use thereof. A medium static magnetic field with an intensity of 0.3T can promote the secretion of granzymes and cytokines of mouse CD8⁺ T cells, increase the level of ATP and mitochondrial respiration, and up-regulate the expression of mitochondrial respiratory chain related genes *Uqcrb* and/or *Ndufs6.* In addition, magnetic receptor candidate genes *Iscal* and *Cryl*/*Cry2* are involved in regulating the expression of *Uqcrb* and/or *Ndufs6.* An in-vivo experiment shows that the static magnetic field can inhibit the growth and development of tumors by means of promoting the secretion of the granzymes and cytokines of the CD8⁺ T cells. A killing ability test shows that the static magnetic field treatment can enhance the killing ability of the CD8⁺ T cells, and infusion of the CD8⁺ T cells treated by means of the magnetic field back into a tumor-grafted mouse has an anti-tumor effect.

## Description

### Technical field

The present invention relates to the field of immune cells, specifically to immune cells treated by magnetic field and uses thereof.

### Background

T cells are an important component of adaptive immunity. T cells can be divided into CD4⁺ T cells and CD8⁺ T cells according to the expression of surface CD4 and CD8 molecules. CD4⁺ T cells, also known as helper T cells, regulate and activate the activity of other immune cells mainly through the release of secreted factors such as IFNγ, IL-4, IL-17, and TNFα. CD8⁺ T cells, also known as killer T cells (CTLs), are capable of directly killing infected, damaged as well as nonfunctional cells and play a key role in antitumor immunity. Killing of tumor target cells by CTLs requires the release of perforin mediated granzymes, particularly granzyme B (granzyme B), which is the major granule component. In addition to this, CTLs also eliminate tumor cells by secreting IFNγ and TNFα.

Magnetic field, like the temperature and pressure present in the environment, is an important physical factor that exists in nature. According to the strength of magnetic field, static magnetic field may be classified as weak magnetic field (< 1 mT), medium magnetic field (1 mT-1 T), strong magnetic field (1-20 T), and ultra-strong magnetic field (> 20 T). Many animals can sense earth's magnetic field (25 µT-65 µT) to navigate and migrate. Cryptochromes (Vry) are recognized as the most likely magnetic receptors, and the protein can photochemically convert free radical pairs to mediate magnetic sensing in birds and Drosophila. In addition, recent studies suggest that the iron sulfur protein Isca1 is also likely to be a magnetic receptor capable of composing rod-like complexes with cryptochrome proteins *in vitro* and, like a compass, changing its own orientation according to the direction of the magnetic field.

In recent years, numerous studies have shown that static magnetic fields have biological effects on cells, such as affecting cell proliferation, differentiation, as well as spindle orientation, among others. From the available studies, it seems that the effects of strong and ultra-strong magnetic fields on normal cells are harmful, such as altering cleavage and spindle orientation, causing DNA damage, etc. However, medium magnetic fields do much less harm to normal cells, and no significant harm from medium magnetic fields has been reported so far. Moreover, more and more studies have shown that medium static magnetic fields have the potential to change cell behavior, such as promoting osteoblast differentiation, inhibiting tumor cell proliferation, promoting oligodendrocyte differentiation and the secretion of neural factors, etc. (Yang J, Zhang J, Ding C, Dong D, Shang P. Regulation of Osteoblast Differentiation and Iron Content in MC3T3-E1 Cells by Static Magnetic Field with Different Intensities. Biological trace element research 2017; Zhang L, Wang J, Wang H, Wang W, Li Z, Liu J, et al. Moderate and strong static magnetic fields directly affect EGFR kinase domain orientation to inhibit cancer cell proliferation. Oncotarget 2016, 7(27): 41527-41539; Prasad A, Teh DBL, Blasiak A, Chai C, Wu Y, Gharibani PM, et al. Static Magnetic Field Stimulation Enhances Oligodendrocyte Differentiation and Secretion of Neurotrophic Factors. Scientific reports 2017, 7(1): 6743.). As the mechanism of magnetic sensing protein based magnetic sensing in animals is revealed, it is possible to manipulate animal and cell behavior by means of magnetic field.

T cells have been reported to respond to static magnetic fields (SMFs). Exposing human T lymphocytes to a strong static magnetic field results in increased apoptosis and damage. Furthermore, exposure of human lymphocytes and macrophages to a 1.5 T static magnetic field inhibits the expression of cytokines IL-6, IL-8, and TNF-α, but IL-10 expression was unchanged. These results indicate that static magnetic fields are able to alter the behavior of T cells. However, the molecular mechanism of how static magnetic fields affect T cell behavior is not clear and is lack of support by *in vivo* data.

### Summary of invention

By treating T cells with a static magnetic field, the inventor finds that the treated T cells produced increased expression or secretion of perforin, granzymes, and/or cytokines, higher ATP levels, and enhanced cell killing activity via the magnetic receptor related genes and mitochondrial respiratory chain related genes.

In the first aspect, the present invention thus provides an activated immune cell characterized by, that the expression or activity of the mitochondrial respiratory chain related genes *Uqcrb* and/or *Ndufs6* is upregulated relative to wild-type immune cells.

In one or more embodiments, the expression or activity of *Uqcrb* is upregulated by at least 20%, at least 30%, at least 40%, at least 50%, at least 60%, at least 70%, or at least 90%.

In one or more embodiments, the expression or activity of *Ndufs6* is upregulated by at least 20%, at least 30%, at least 40%, at least 50%, at least 60%, at least 70%, or at least 90%.

In one or more embodiments, the immune cells are treated with a magnetic field.

In one or more embodiments, the magnetic field is a static magnetic field.

In one or more embodiments, the magnetic field is a static magnetic field with a strength of 1 mT-1.0 T. Preferably, the strength of the magnetic field is 0.05-0.9 T, 0.1-0.8 T, 0.2-0.7 T, 0.3-0.6 T or 0.4-0.5 T.

In one or more embodiments, the treatment is sujecting the cells to the magnetic field for at least 48 h, at least 60 h, at least 72 h, at least 84 h, at least 96 h, or at least 108 h.

In one or more embodiments, the magnetic receptor related genes of the immune cell, Isca1, Cry1 and/or Cry2, are involved in regulating the expression or activity of the mitochondrial respiratory chain related genes Uqcrb and/or Ndufs6.

In one or more embodiments, the immune cells have an increased expression or secretion of perforin, granzymes, and/or cytokines.

In one or more embodiments, the immune cells have elevated levels of ATP.

In one or more embodiments, the immune cells are selected from the group consisting of: lymphocytes, dendritic cells, monocytes/macrophages, granulocytes, and mast cells.

In one or more embodiments, the immune cells are T cells, preferably CD8⁺ T cells or CD4⁺ T cells.

In one or more embodiments, the cell killing activity of the immune cells is enhanced.

In another aspect, the invention provides a pharmaceutical composition comprising the immune cells described herein and a pharmaceutically acceptable carrier.

In one or more embodiments, the immune cells are selected from the group consisting of: lymphocytes, dendritic cells, monocytes/macrophages, granulocytes, and mast cells.

In one or more embodiments, the immune cells are T cells, preferably CD8⁺ T cells or CD4⁺ T cells.

In another aspect, the invention provides use of a magnetic field in upregulating the expression or activity of mitochondrial respiratory chain related genes of a cell, or elevating the ATP level of a cell.

In one or more embodiments, the magnetic field is a static magnetic field.

In one or more embodiments, the magnetic field is a static magnetic field with a strength of 1 mT-1.0 T. Preferably, the strength of the magnetic field is 0.05-0.9 T, 0.1-0.8 T, 0.2-0.7 T, 0.3-0.6 T or 0.4-0.5 T.

In one or more embodiments, the cells are immune cells.

In one or more embodiments, the immune cells are selected from the group consisting of: lymphocytes, dendritic cells, monocytes/macrophages, granulocytes, and mast cells.

In one or more embodiments, the immune cells are T cells, preferably CD8⁺ T cells or CD4⁺ T cells.

In one or more embodiments, the mitochondrial respiratory chain related genes are *Uqcrb* and/or *Ndufs6.* In one or more embodiments, the expression or activity of *Uqcrb* is upregulated by at least 20%, at least 30%, at least 40%, at least 50%, at least 60%, at least 70%, or at least 90%. In one or more embodiments, the expression or activity of *Ndufs6* is upregulated by at least 20%, at least 30%, at least 40%, at least 50%, at least 60%, at least 70%, or at least 90%.

In one or more embodiments, the cells have unchanged expression of the magnetic receptor related genes Isca1, Cry1, and/or Cry2.

In one or more embodiments, the cells are placed in the magnetic field for at least 48 h, at least 60 h, at least 72 h, at least 84 h, at least 96 h, or at least 108 h.

In another aspect, the invention provides use of a magnetic field in promoting the expression or secretion of perforin, granzymes and/or cytokines of cells, or in increasing the cell killing activity of cells.

In one or more embodiments, the magnetic field is a static magnetic field.

In one or more embodiments, the magnetic field is a static magnetic field with a strength of 1 mT-1.0 T. Preferably, the strength of the magnetic field is 0.05-0.9 T, 0.1-0.8 T, 0.2-0.7 T, 0.3-0.6 T or 0.4-0.5 T.

In one or more embodiments, the cells are immune cells.

In one or more embodiments, the immune cells are selected from the group consisting of: lymphocytes, dendritic cells, monocytes/macrophages, granulocytes, and mast cells.

In one or more embodiments, the immune cells are T cells, preferably CD8⁺ T cells or CD4⁺ T cells.

In one or more embodiments, the magnetic receptor related genes of the cell, Isca1, Cry1 and/or Cry2, are involved in regulating the expression of the mitochondrial respiratory chain related genes *Uqcrb* and/or *Ndufs6.*

In one or more embodiments, the cells have upregulated expression or activity of the mitochondrial respiratory chain related genes *Uqcrb* and/or *Ndufs6.* In one or more embodiments, the expression or activity of *Uqcrb* is upregulated by at least 20%, at least 30%, at least 40%, at least 50%, at least 60%, at least 70%, or at least 90%. In one or more embodiments, the expression or activity of *Ndufs6* is upregulated by at least 20%, at least 30%, at least 40%, at least 50%, at least 60%, at least 70%, or at least 90%.

In one or more embodiments, the cells have unchanged expression of the magnetic receptor related genes Isca1, Cry1, and/or Cry2.

In one or more embodiments, the cells are placed in the magnetic field for at least 48 h, at least 60 h, at least 72 h, at least 84 h, at least 96 h, or at least 108 h.

In another aspect, the invention provides use of magnetic receptor related genes of cells in response to a magnetic field in increasing the expression or activity of mitochondrial respiratory chain related gene of the cells, or in increasing the ATP level of the cells.

In one or more embodiments, the expression or activity of a magnetic receptor related gene in response to a magnetic field is unchanged.

In one or more embodiments, the magnetic field is a static magnetic field.

In one or more embodiments, the magnetic field is a static magnetic field with a strength of 1 mT-1.0 T. Preferably, the strength of the magnetic field is 0.05-0.9 T, 0.1-0.8 T, 0.2-0.7 T, 0.3-0.6 T or 0.4-0.5 T.

In one or more embodiments, the magnetic receptor related genes respond to a magnetic field when the cells are placed in the magnetic field for at least 48 h, at least 60 h, at least 72 h, at least 84 h, at least 96 h, or at least 108 h.

In one or more embodiments, the cells are immune cells.

In one or more embodiments, the immune cells are selected from the group consisting of: lymphocytes, dendritic cells, monocytes/macrophages, granulocytes, and mast cells.

In one or more embodiments, the immune cells are T cells, preferably CD8⁺ T cells or CD4⁺ T cells.

In one or more embodiments, the mitochondrial respiratory chain related genes are *Uqcrb* and or *Ndufs6.*

In one or more embodiments, the magnetic receptor related genes are Isca1, Cry1, and/or Cry2.

In another aspect, the invention provides use of upregulating the expression or activity of mitochondrial respiratory chain related genes in promoting the expression or secretion of perforin, granzymes and/or cytokines of cells, or in improving the cell killing activity of cells.

In one or more embodiments, the cells are immune cells.

In one or more embodiments, the immune cells are selected from the group consisting of: lymphocytes, dendritic cells, monocytes/macrophages, granulocytes, and mast cells.

In one or more embodiments, the immune cells are T cells, preferably CD8⁺ T cells or CD4⁺ T cells.

In one or more embodiments, the mitochondrial respiratory chain related genes are *Uqcrb* and/or *Ndufs6.* In one or more embodiments, the expression or activity of *Uqcrb* is upregulated by at least 20%, at least 30%, at least 40%, at least 50%, at least 60%, at least 70%, or at least 90%. In one or more embodiments, the expression or activity of Ndufs6 is upregulated by at least 20%, at least 30%, at least 40%, at least 50%, at least 60%, at least 70%, or at least 90%.

In one or more embodiments, the granzymes are granzymes A and/or B.

In one or more embodiments, the cytokine comprises one or more selected from the group consisting of interleukin (IL), colony-stimulating factor (CSF), interferon (IFN), tumor necrosis factor (TNF), transforming growth factor-β Family (TGF-β), growth factors (GFS), and chemokine family.

In another aspect, the invention provides use of the magnetic field described herein, an agent that upregulates the expression or activity of the mitochondrial respiratory chain related genes in the preparation of an agent containing immune cells or in the preparation of an agent that enhances the killing activity of immune cells.

In one or more embodiments, the magnetic field is a static magnetic field.

In one or more embodiments, the magnetic field is a static magnetic field with a strength of 1 mT-1.0 T. Preferably, the strength of the magnetic field is 0.05-0.9 T, 0.1-0.8 T, 0.2-0.7 T, 0.3-0.6 T or 0.4-0.5 T.

In one or more embodiments, the immune cells are placed in the magnetic field for at least 48 h, at least 60 h, at least 72 h, at least 84 h, at least 96 h, or at least 108 h.

In one or more embodiments, the immune cells have elevated levels of ATP.

In one or more embodiments, the immune cells have elevated expression or secretion of perforin, granzymes, and/or cytokines.

In one or more embodiments, the cell killing activity of the immune cells is increased.

In one or more embodiments, the immune cells are selected from the group consisting of: lymphocytes, dendritic cells, monocytes/macrophages, granulocytes, and mast cells.

In one or more embodiments, the immune cells are T cells, preferably CD8⁺ T cells or CD4⁺ T cells.

In one or more embodiments, the agent that upregulates the expression or activity of a mitochondrial respiratory chain related gene is a primer that recognizes a mitochondrial respiratory chain related gene or a vector that includes the coding sequence of a mitochondrial respiratory chain related gene. Preferably, the mitochondrial respiratory chain related genes are *Uqcrb* and/or *Ndufs6.* In one or more embodiments, the expression or activity of *Uqcrb* is upregulated by at least 20%, at least 30%, at least 40%, at least 50%, at least 60%, at least 70%, or at least 90%. In one or more embodiments, the expression or activity of Ndufs6 is upregulated by at least 20%, at least 30%, at least 40%, at least 50%, at least 60%, at least 70%, or at least 90%.

In one or more embodiments, the sequences of the primers that recognize the mitochondrial respiratory chain related genes comprises one or more sequences selected from the group consisting of SEQ ID Nos: 9,10,15,16. Preferably, the sequences of the primers are SEQ ID Nos: 9 and 10 or SEQ ID Nos: 15 and 16.

In another aspect, the invention provides use of the magnetic field described herein, an agent that upregulates the expression or activity of mitochondrial respiratory chain related genes, and immune cells in the preparation of a medicament for the treatment of cancer.

In one or more embodiments, the magnetic field is a static magnetic field.

In one or more embodiments, the magnetic field is a static magnetic field with a strength of 1 mT-1.0 T. Preferably, the strength of the magnetic field is 0.05-0.9 T, 0.1-0.8 T, 0.2-0.7 T, 0.3-0.6 T or 0.4-0.5 T.

In one or more embodiments, the medicament comprises T cells placed in the magnetic field for at least 48 h, at least 60 h, at least 72 h, at least 84 h, at least 96 h, or at least 108 h.

In one or more embodiments, the cancer is a cancer targeted by CD8⁺ T cells, including solid tumors and hematological tumors, such as adenocarcinomas, lung, colon, colorectal, breast, ovarian, cervical, gastric, cholangiocarcinomas, gallbladder, esophageal, pancreatic, and prostate cancers, and leukemias and lymphomas, such as B cell lymphomas, mantle cell lymphomas, acute lymphoblastic leukemias, chronic lymphocytic leukemia, hairy cell leukemia, and acute myeloid leukemia, among others.

In one or more embodiments, the cancer is melanoma and/or breast cancer.

In one or more embodiments, the T cells are said to have elevated levels of ATP.

In one or more embodiments, the T cells have elevated expression or secretion of perforin, granzymes, and/or cytokines.

In one or more embodiments, the cell killing activity of said T cells is increased.

In one or more embodiments, the immune cells are selected from the group consisting of: lymphocytes, dendritic cells, monocytes/macrophages, granulocytes, and mast cells.

In one or more embodiments, the immune cells are T cells, preferably CD8⁺ T cells or CD4⁺ T cells.

In one or more embodiments, an agent that upregulates the expression or activity of a mitochondrial respiratory chain related gene is a primer that recognizes a mitochondrial respiratory chain related gene or a vector that includes the coding sequence of a mitochondrial respiratory chain related gene. Preferably, mitochondrial respiratory chain related genes are *Uqcrb* and/or *Ndufs6.* In one or more embodiments, the expression or activity of *Uqcrb* is upregulated by at least 20%, at least 30%, at least 40%, at least 50%, at least 60%, at least 70%, or at least 90%. In one or more embodiments, the expression or activity of *Ndufs6* is upregulated by at least 20%, at least 30%, at least 40%, at least 50%, at least 60%, at least 70%, or at least 90%.

In one or more embodiments, the sequence of the primer that recognizes the mitochondrial respiratory chain related gene comprises one or more sequences selected from the group consisting of SEQ ID Nos: 9, 10, 15, and 16. Preferably, the sequences of the primers are SEQ ID Nos: 9 and 10 or SEQ ID Nos: 15 and 16.

Methods for the preparation of immune cells are also provided herein, comprising one or more steps selected from (1)-(4):
(1) treating immune cells with the magnetic field described herein,
(2) treating a subject containing the immune cells with the magnetic field described herein,
(3) upregulating the expression and/or activity of mitochondrial respiratory chain related genes of immune cells.

In one or more embodiments, the magnetic field is a static magnetic field.

In one or more embodiments, the magnetic field is a static magnetic field with a strength of 1 mT-1.0 T. Preferably, the strength of the magnetic field is 0.05-0.9 T, 0.1-0.8 T, 0.2-0.7 T, 0.3-0.6 T or 0.4-0.5 T.

In one or more embodiments, the treatment is subjecting the immune cells or subject to the magnetic field for at least 48 h, at least 60 h, at least 72 h, at least 84 h, at least 96 h, or at least 108 h.

In one or more embodiments, an agent that upregulates the expression or activity of a mitochondrial respiratory chain related gene is a primer that recognizes a mitochondrial respiratory chain related gene or a vector that includes the coding sequence of a mitochondrial respiratory chain related gene. Preferably, the mitochondrial respiratory chain related genes are *Uqcrb* and/or *Ndufs6.* In one or more embodiments, the expression or activity of *Uqcrb* is upregulated by at least 20%, at least 30%, at least 40%, at least 50%, at least 60%, at least 70%, or at least 90%. In one or more embodiments, the expression or activity of *Ndufs6* is upregulated by at least 20%, at least 30%, at least 40%, at least 50%, at least 60%, at least 70%, or at least 90%.

In one or more embodiments, the immune cells are selected from the group consisting of: lymphocytes, dendritic cells, monocytes/macrophages, granulocytes, and mast cells.

In one or more embodiments, the immune cells are T cells, preferably CD8⁺ T cells or CD4⁺ T cells.

In another aspect, the invention provides a method for increasing the ATP level of a cell *in vivo* or *in vitro,* promoting mitochondrial respiration of a cell *in vivo* or *in vitro,* and enhancing the killing activity of immune cells *in vivo* or *in vitro,* comprising one or more steps selected from (1)-(3):
(1) treating immune cells with the magnetic field described herein,
(2) treating a subject containing immune cells with the magnetic field described herein,
(3) upregulating the expression and/or activity of mitochondrial respiratory chain related genes of immune cells,

In one or more embodiments, the mitochondrial respiratory chain related genes are *Uqcrb* and/or *Ndufs6.* In one or more embodiments, the expression or activity of *Uqcrb* is upregulated by at least 20%, at least 30%, at least 40%, at least 50%, at least 60%, at least 70%, or at least 90%. In one or more embodiments, the expression or activity of *Ndufs6* is upregulated by at least 20%, at least 30%, at least 40%, at least 50%, at least 60%, at least 70%, or at least 90%.

In one or more embodiments, the magnetic receptor related genes are Isca1, Cry1, and/or Cry2.

In one or more embodiments, the magnetic field is a static magnetic field.

In one or more embodiments, the magnetic field is a static magnetic field with a strength of 1 mT-1.0 T. Preferably, the strength of the magnetic field is 0.05-0.9 T, 0.1-0.8 T, 0.2-0.7 T, 0.3-0.6 T or 0.4-0.5 T.

In one or more embodiments, the treatment is subjecting the immune cells or subject to the magnetic field for at least 48 h, at least 60 h, at least 72 h, at least 84 h, at least 96 h, or at least 108 h.

In one or more embodiments, the immune cells are selected from the group consisting of: lymphocytes, dendritic cells, monocytes/macrophages, granulocytes, and mast cells.

In one or more embodiments, the immune cells are T cells, preferably CD8⁺ T cells or CD4⁺ T cells.

In another aspect, the invention provides a method for treating cancer comprising administering to a subject in need a therapeutically effective amount of an immune cell described herein, or a pharmaceutical composition described herein.

In another aspect, the present invention provides a method of treating cancer comprising treating a subject with a magnetic field.

In one or more embodiments, the magnetic field is a static magnetic field.

In one or more embodiments, the magnetic field is a static magnetic field with a strength of 1 mT-1.0 T. Preferably, the strength of the magnetic field is 0.05-0.9 T, 0.1-0.8 T, 0.2-0.7 T, 0.3-0.6 T or 0.4-0.5 T.

In one or more embodiments, the treatment is placing the immune cells or subject in the magnetic field for at least 48 h, at least 60 h, at least 72 h, at least 84 h, at least 96 h, or at least 108 h.

In one or more embodiments, the cancer is a cancer targeted by CD8⁺ T cells, including solid tumors and hematological tumors, such as adenocarcinomas, lung, colon, colorectal, breast, ovarian, cervical, gastric, cholangiocarcinomas, gallbladder, esophageal, pancreatic, and prostate cancers, and leukemias and lymphomas, such as B cell lymphomas, mantle cell lymphomas, acute lymphoblastic leukemias, chronic lymphocytic leukemia, hairy cell leukemia, and acute myeloid leukemia, melanoma, among others.

In one or more embodiments, the cancer is melanoma and/or breast cancer.

In one or more embodiments, the immune cells are selected from the group consisting of: lymphocytes, dendritic cells, monocytes/macrophages, granulocytes, and mast cells.

In one or more embodiments, the immune cells are T cells, preferably CD8⁺ T cells or CD4⁺ T cells.

In another aspect, the invention provides a magnetic part comprising a magnet loaded body.

In one or more embodiments, the magnet is a button magnet of 0.3 T or 0.6 T. In one or more embodiments, the body is a resin fibreboard.

In one or more embodiments, the body is a rectangular plate.

In one or more embodiments, the body is 0.1-5.0 cm, preferably 1.5 cm thick.

In one or more embodiments, the magnet is 0.5-5 cm, preferably 1 cm in diameter.

In one or more embodiments, each two magnets are separated by 0.5-5 cm, preferably 2.5 cm.

In one or more embodiments, the magnet is mosaic within the body.

In another aspect, the invention provides a device which is a mammalian cage comprising the magnetic parts described herein.

In one or more embodiments, the magnetic part is located anywhere in the cage, such as at the top, bottom, side, center, preferably at the bottom.

In one or more embodiments, the magnetic part has the N-pole facing up and the S-pole facing down.

In another aspect, the invention provides a method for preparing the device described herein, comprising placing the magnetic parts described herein at the top, bottom, side, central position of a mammalian cage, preferably at the bottom.

In another aspect, the invention provides use of the magnetic part or device described herein in the preparation of an immune cell containing reagents or drugs for the treatment of cancer.

In one or more embodiments, the immune cells are selected from the group consisting of: lymphocytes, dendritic cells, monocytes/macrophages, granulocytes, and mast cells. In one or more embodiments, the immune cells are T cells, preferably CD8⁺ T cells or CD4⁺ T cells.

In another aspect, the invention provides use of the magnetic parts or device described herein in increasing ATP levels of cells *in vivo* or *in vitro,* in promoting mitochondrial respiration of cells *in vivo* or *in vitro,* and in enhancing killing activity of immune cells *in vivo* or *in vitro.* In one or more embodiments, the immune cells are selected from the group consisting of: lymphocytes, dendritic cells, monocytes/macrophages, granulocytes, and mast cells. In one or more embodiments, the immune cells are T cells, preferably CD8⁺ T cells or CD4⁺ T cells.

In another aspect, the invention provides use of the magnetic parts or device described herein in the treatment of cancer.

The beneficial effects of the present invention lie in:
(1) It is revealed that a moderate static magnetic field promotes the secretion of granzymes and cytokines from immune cells, such as T cells, and ATP and mitochondrial respiration levels by regulating the expression of mitochondrial respiratory chain genes.
(2) New links between magnetic receptor candidate genes and mitochondrial respiratory chain genes are established, which provide new clues for understanding how cells receive magnetic signals and then translate into biological signals to elicit biological effects. Furthermore, our study establishes a novel link between magnetic fields, mitochondrial ATP synthesis, and immune cell (e.g., T cell) killing, which reveals not only a novel immunophysiological effect but also a novel mechanism of immune cell killing by a magnetic field.
(3) It is confirmed that a moderate static magnetic field enhances the antitumor effect of immune cells (e.g., T cells) and that reinfusion of magnetic field treated CTLs cells *in vivo* has a significant antitumor effect. This developes a new physical approach based on magnetic field sensing to enhance the antitumor effect of immune cells. In contrast to existing T cell (CAR-T) therapies with chimeric antigens, the method does not require the transfer of DNA fragments into target cells by means of gene editing, which is a noninvasive means of manipulating cellular function.
(4) Different experimental parameters such as cell type, magnetic field strength, exposure time, etc., lead to different cellular effects. In this study, we examine the cellular effects of a moderate static magnetic field with different magnetic field strengths and different TCR stimulation periods on immune cells (e.g., T cells) *in vitro,* confirming that the moderate static magnetic field for 72 h of TCR stimulation can enhance the secretion of granzymes and cytokines by immune cells. Furthermore, *in vivo* experiments confirm that the moderate static magnetic field (0.6 T magnetic plate treatment) promotes the secretion of granzymes and cytokines from tumor infiltrating immune cells.

### Description of drawings

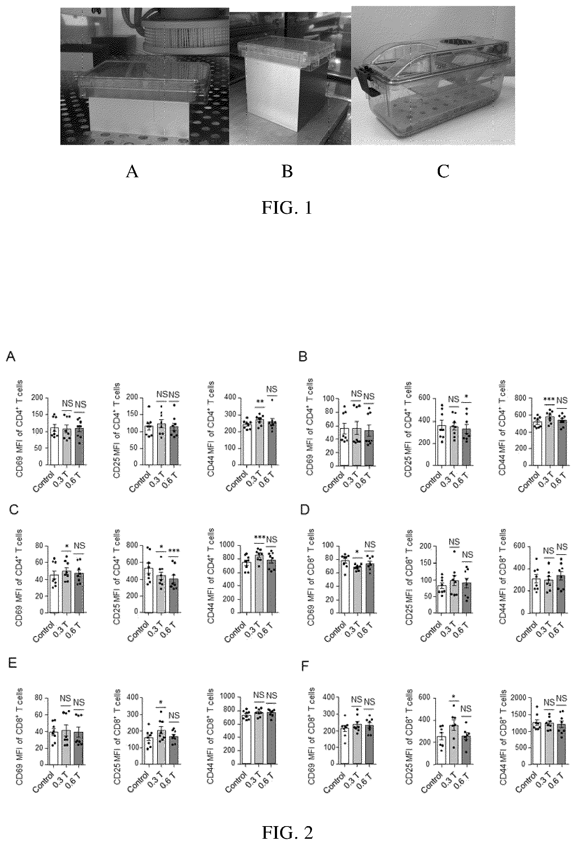

Fig. 1 shows the permanent magnet and magnetic plate used experimentally. (A) 0.3 T permanent magnet for *in vitro* experiments, (B) 0.6 T permanent magnet for *in vitro* experiments, and (C) the magnetic plate for *in vivo* experiments were placed on the bottom of the mouse cage.
Fig. 2 shows the effect of a moderate static magnetic field on the surface activation molecule expression of T cells *in vitro.* (A-C) CD4⁺ T cells were stimulated with anti-CD3 and anti-CD28 antibodies *in vitro* for 24 h (A), 48 h (B) and 72 h (C), after that the positive ratios of CD69, CD25 and CD44 were determined by flow cytometric analysis (n = 8). (D-F) CD8⁺ T cells were stimulated with anti-CD3 and anti-CD28 antibodies *in vitro* for 24 h (A), 48 h (B) and 72 h (C), after that the positive ratios of CD69, CD25 and CD44 were determined by flow cytometric analysis (n = 8). Cell samples were treated with a 0.3 T or 0.6 T magnet, and control cells were not treated with a magnet.

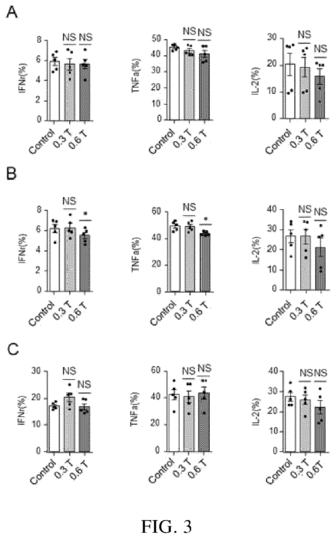

Fig. 3 shows that a moderate static magnetic field had no significant effect on cytokine secretion by CD4⁺ T cells *in vitro.* CD4⁺ T cells were stimulated with anti-CD3 and anti-CD28 antibodies *in vitro* for 24 h (A), 48 h (B) and 72 h (C), after the secretion of IFNγ, TNFα and IL-2 was detected using flow cytometric analysis, the comparative analysis of the proportion of cells with positive expression was conducted. Cell samples were treated with a 0.3 T or 0.6 T magnet, and control cells were not treated with a magnet.

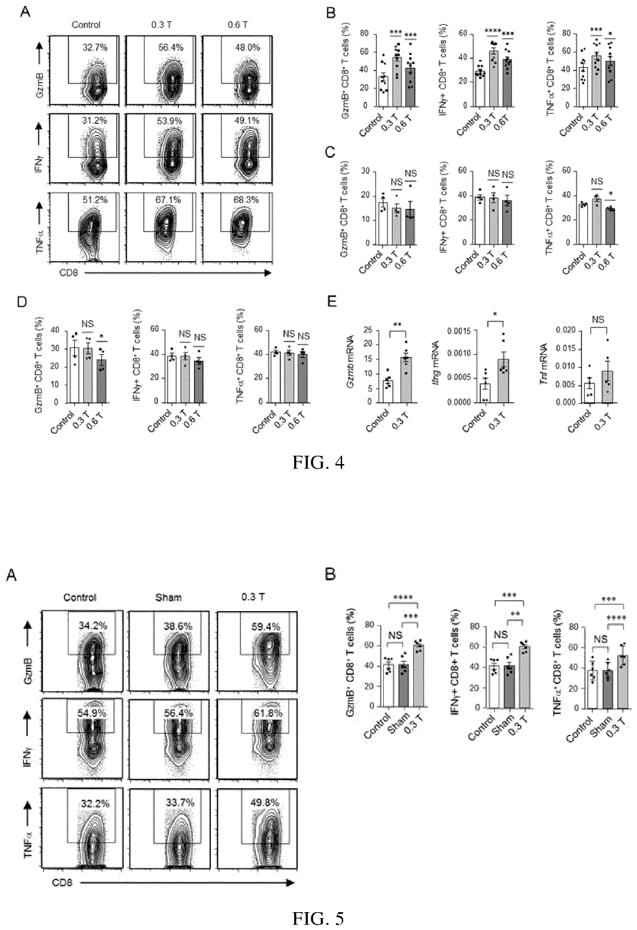

Fig. 4 shows that a moderate static magnetic field enhances the secretion of granzymes and cytokines by CD8⁺ T cells. (A) Granzyme and cytokine secretion levels of mouse CD8⁺ T cells were determined by flow cytometry. Cell samples were stimulated *in vitro* with anti-CD3 and anti-CD28 antibodies and treated with a 0.3 T or 0.6 T permanent magnet, while control cells were not treated with a magnet. (B-D) Statistics of the proportion of CD8⁺ T cells that express GzmB, IFNγ and TNFα,: 72 h (B, n = 10), 24 h (C, n = 4), and 48 h (D, n = 4). (E) Comparative analysis of the transcription levels of *Gmzb, Tnfα,* and *Ifng* gene in CD8⁺ T cells (n = 6). The transcription levels of all target genes were normalized according to the internal reference β-actin.
Fig. 5 shows that the pseudotyped material had no significant effect on cytokine secretion by CD8⁺ T cells. (A) Secretion levels of granzyme and cytokine by mouse CD8⁺ T cells were determined by flow cytometry. Cell samples were stimulated *in vitro* with anti-CD3 and anti-CD28 antibodies and treated with metallic material without magnetism (artefacts) or a 0.3 T permanent magnet (0.3 T), while control cells were not treated with a magnet. (B) Statistics of the proportion of CD8⁺ T cells that express GzmB, IFNγ and TNFα (n = 6).

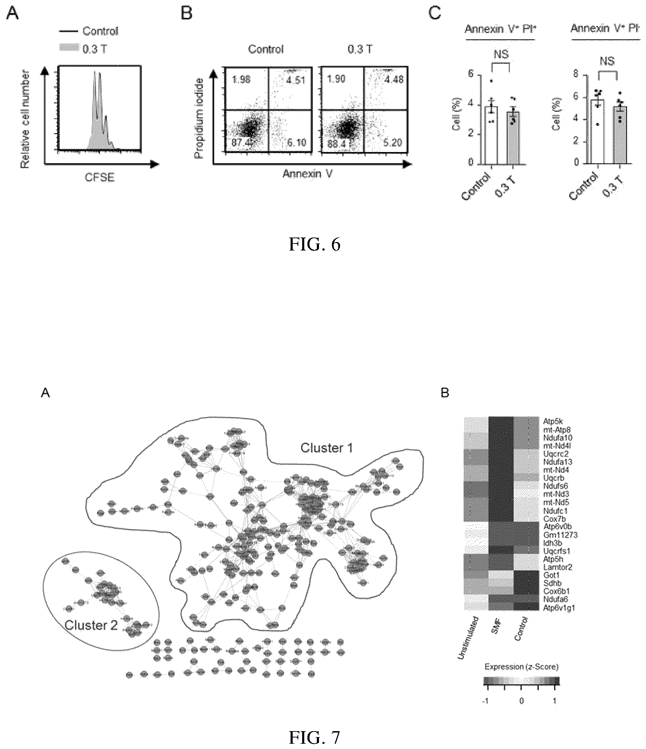

Fig. 6 shows that a moderate static magnetic field has no significant effect on CD8⁺ T cell proliferation and apoptosis *in vitro.* CD8 + T cells were stimulated with anti-CD3 and anti-CD28 antibodies for 72 h, and CFSE expression was measured by flow analysis to indicate cell proliferation (A); detection of annexin V and PI (propidium iodide) indicates apoptosis (B). (C) Statistics of the proportion of each cell stained by annexin V and PI for CD8⁺ T cells. Cell samples were treated with a 0.3 T magnet, and control cells were not treated with a magnet.
Fig. 7 shows that a moderate static magnetic field promotes the expression of mitochondrial respiratory chain related genes. (A) PPI network plot of genes differentially expressed by CD8⁺ T cells treated with a 0.3 T magnet versus magnet untreated control cells (protein interaction map). (B) Gene expression heatmap of TCR stimulated CD8⁺ T cells (i.e. SMF) treated with a 0.3 T magnet, TCR stimulated control cells without magnet treatment, and TCR stimulated cells.

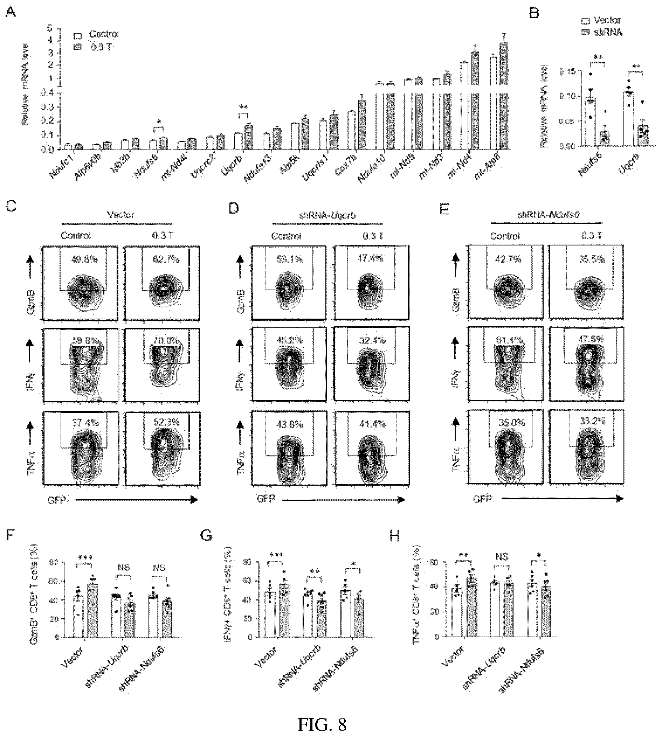

Fig. 8 shows that a moderate static magnetic field promotes granzyme and cytokine secretion by CD8⁺ T cell by upregulating mitochondrial ATP synthesis related respiratory chain genes. (A) Comparative analysis of transcription and expression levels of mitochondrial ATP synthesis related respiratory chain genes in CD8⁺ T cells. Cell samples were stimulated with anti-CD3 and anti-CD28 antibodies for 72 h *in vitro* and treated with a 0.3 T permanent magnet, while control cells were not treated with a magnet (n = 5-7). (B) *Uqcrb* or *Ndufs6* knockdown in CD8⁺ T cells detect the transcription levels before and after knockdown (n = 5). The transcription levels of all target genes were normalized according to the internal reference β-actin. (C-E) Flow cytometry was used to determine granzyme and cytokine secretion levels in knockdown CD8⁺ T cells transfected with empty vector (panel C), shRNA-Uqcrb (panel D), and shRNA-Ndufs6 (panel E). (F-H) Statistics of the proportion of knockdown CD8⁺ T cells that express gzmB, IFNγ and TNFα cells (n = 5-7).

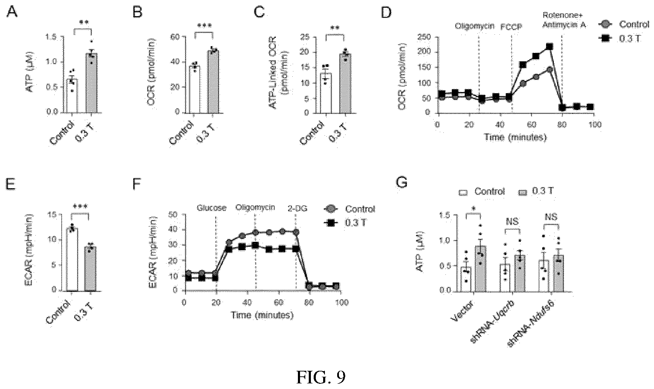

Fig. 9 shows that a moderate static magnetic field enhances the levels of ATP production and mitochondrial oxygen consumption in CD8⁺ T cells. (A) Detection of intracellular ATP levels in CD8⁺ T cells. (B) Mitochondrial basal oxygen consumption rate (baseline OCR) in CD8⁺ T cells (n = 4). (C) Mitochondrial ATP synthesis related OCR in CD8⁺ T cells (baseline OCR minus OCR after addition of oligomycin) (n = 4). (D) Diagram of mitochondrial OCR tests of CD8⁺ T cells with added inhibitors of oligomycin, FCCP (2,4-dinitrophenol), rotenone as well as antimycin A. (B) Mitochondrial extracellular acid producing capacity (Baseline ECAR) of CD8⁺ T cells (n = 4). (E) Diagram of mitochondrial ECAR test for CD8⁺ T cells with added inhibitors of glucose, oligomycin, and 2-DG (2-deoxy-D-glucose). (G) ATP concentration in knockdown CD8⁺ T cells (n = 5). The above cell samples were stimulated with anti-CD3 and anti-CD28 antibodies *in vitro* for 72 h and treated with a 0.3 T permanent magnet, while the control cells were not treated with a magnet.

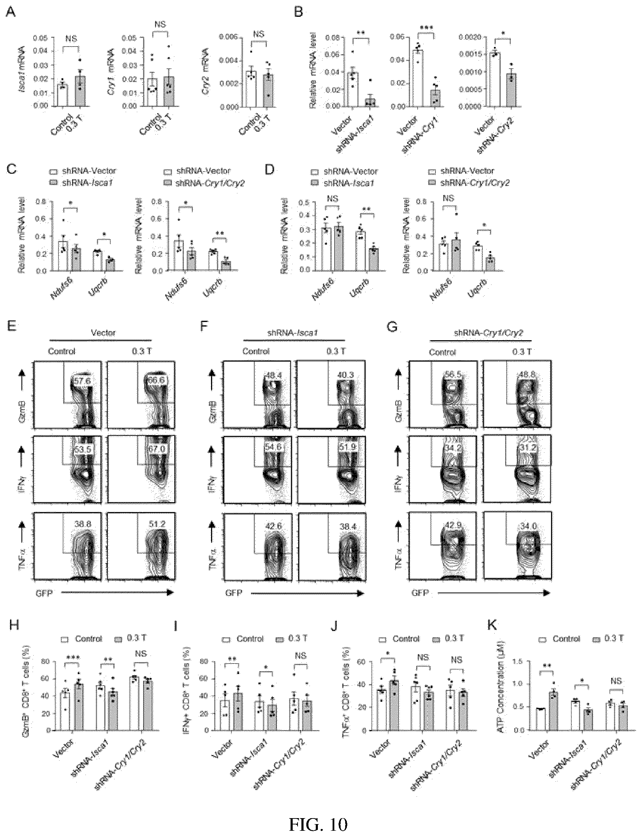

Fig. 10 shows that the magnetic receptor candidate genes are involved in regulating the expression of *Uqcrb* and *Ndufs6.* (A) Comparative analysis of transcription and expression levels of *Iscal* and *Cry1* in CD8⁺ T cells (n = 4-6). (B) The transcription levels before and after knockdown of *Iscal, Cry1*, or *Cry2* in CD8⁺ T cells (n = 4-5). (C-D) CD8⁺ T cells were tested for changes in mRNA levels of *Uqcrb* and *Ndufs6* in the presence of knockdown Isca1 or Cry1/Cry2, (C) no magnet treatment; (D) 0.3 T magnet treatment (n = 5). The transcription levels of all target genes were normalized according to the internal reference β-actin. (E-G) Granzyme and cytokine secretion levels in knockdown CD8⁺ T cells transfected with empty vector (panel E), shRNA-Iscal (panel F), and shRNA-Cry1/Cry2 (panel G) were determined by flow cytometry. (H-J) Statistics of the proportion of knockdown CD8⁺ T cells that express GzmB, IFNγ and TNFα (n = 5-6). (K) ATP concentration in knockdown CD8⁺ T cells (n = 4).

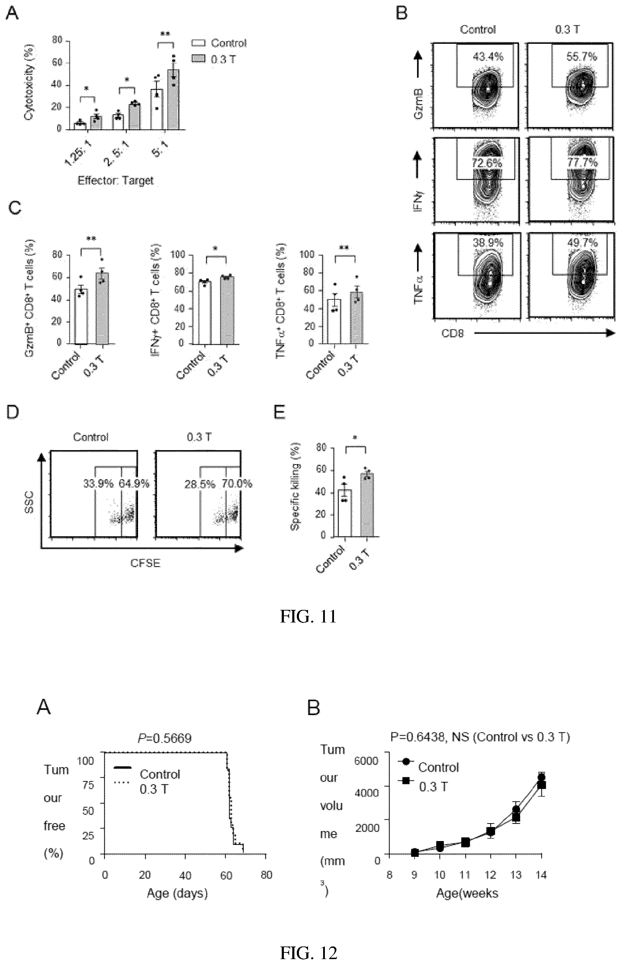

Fig. 11 shows that a moderate static magnetic field enhances the killing ability of CTLs cells. (A) The killing ability of CTLs cells obtained from OT-I mice was indicated by detecting the release of LDH (n = 4). (B) The secretion of granzyme and cytokines by CTLs was detected by flow cytometry. (C) Statistics of the proportion of CTL cells that express GzmB, IFNγ and TNFα (n = 4). (D) The killing efficiency *in vivo* was detected by flow cytometry. CTL cells were obtained from OT-I transgenic mice and treated with a 0.3 T magnet, and control cells were not treated with a magnet. CTLs cells were injected into C57BL/6 recipient mice via tail vein, followed by injection of CFSE labeled mouse spleen cells (CFSE^{low} labeled spleen cells incubated with OVA2₅₇₋₂₆₄ were mixed 1:1 with CFSE^{high} labeled spleen cells not incubated with OVA₂₅₇₋₂₆₄), and finally, the spleens of recipient mice were harvested and analyzed for CFSE expression by flow cytometry to detect killing efficiency. (E) Comparative analysis of killing efficiency for *in vivo* killing experiments.
Fig. 12 shows that the 0.3T magnetic plate treatment did not significantly affect tumor initiation and growth. PyMT mice were treated with a 0.3 T magnetic plate, and control mice were treated with a blank plate without a magnet. Time to tumor onset (A; n = 11) as well as tumor growth (B; n = 7) were monitored.

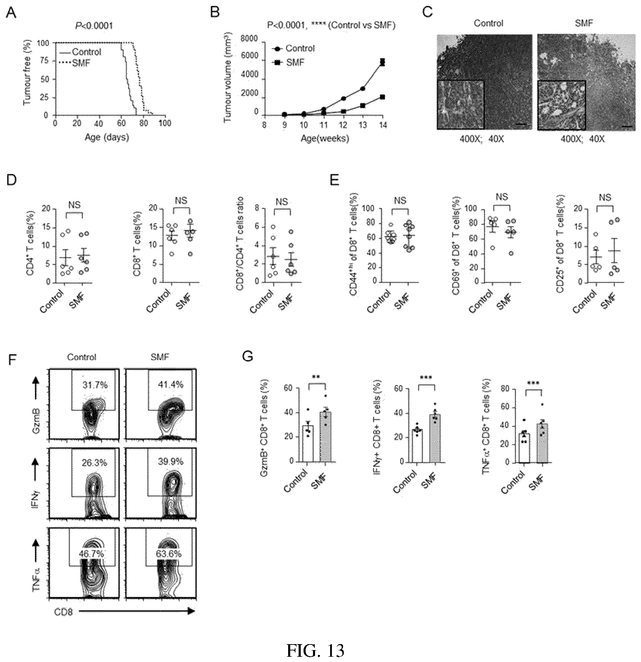

Fig. 13 shows that a moderate static magnetic field promotes antitumor responses of CD8⁺ T cells *in vivo.* PyMT mice were treated with a 0.6 T magnetic plate, whereas control mice were treated with a blank plate containing no magnet. Time to tumor onset (A; n = 30) as well as tumor growth (B; n = 17) were monitored. (C) HE staining results of breast tumor tissue sections in PyMT mice (magnification, ×40 and ×400; scale bar, 200 mm). (D) Analysis of tumor infiltrating CD4⁺ T cells, CD8⁺ T cells as well as the ratio of CD8⁺/CD4⁺ T cells in PyMT mice (n = 6) and results of cell ratio were obtained from flow cytometry analysis. (E) Analysis of the proportion of tumor infiltrating CD8+ T cells positive for CD69, CD44 and CD25 expression in PyMT mice (n = 5). (F) Granzyme and cytokine secretion by tumor infiltrating CD8⁺ T cells in PyMT mice. (G) Statistics of the proportion of tumor infiltrating CD8⁺ T cells that express gzmB, IFNγ and TNFα cells (n = 5-6). "SMF" in the figure represents magnetic field treated mice.

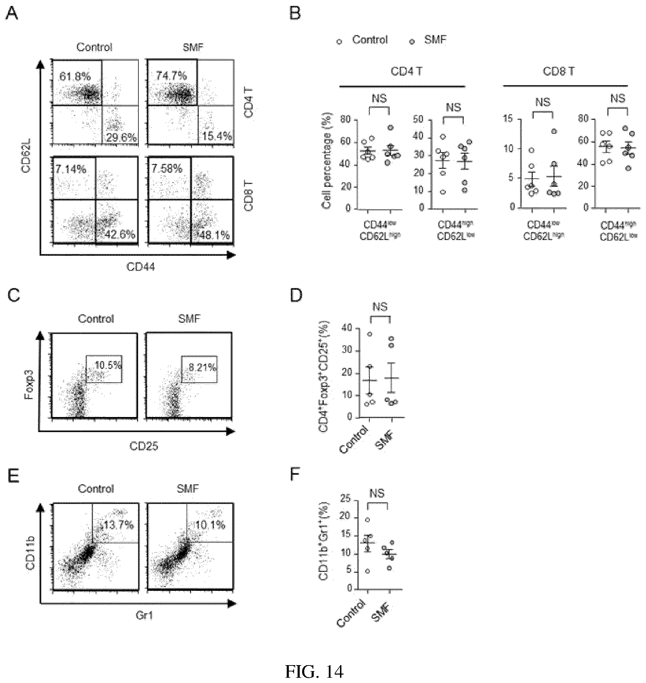

Fig. 14 shows that a moderate static magnetic field does not affect tumor infiltrating CD8⁺ T cell subset distribution. (A) Flow cytometric analysis of CD62L and CD44 expression on tumor infiltrating T cells in PyMT mice. (B) Statistics of the proportion of CD44^{low} CD62E^{high} and CD44^{high} CD62E^{low} cells (n = 6). (C) The proportion of tumor infiltrating Treg cells (CD4⁺CD25⁺ Foxp3⁺) in PyMT mice was determined by flow cytometry. (D) Statistical analysis of the proportion of Treg cells (n = 5). (E) The proportion of tumor MDSC cells (Gr1⁺ CD11b⁺) in PyMT mice was determined by flow cytometry. (F) Statistical analysis of the proportion of MDSC cells (n = 5).

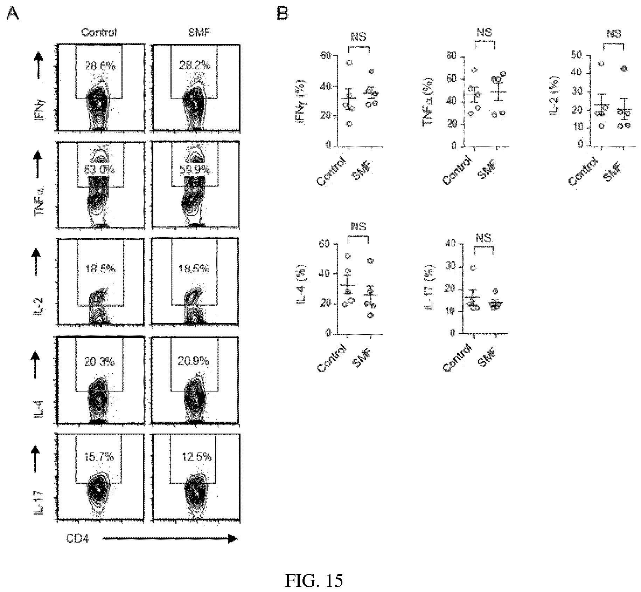

Fig. 15 shows that a moderate static magnetic field does not affect the secretion of cytokines by tumor infiltrating CD4⁺ T cells. (A) Cytokine secretion of tumor infiltrating CD4⁺ T cells in PyMT mice was determined by flow cytometry. (B) Statistical analysis of the proportion of the cytokine secretion positive population of tumor infiltrating CD4⁺ T cells (n = 5).

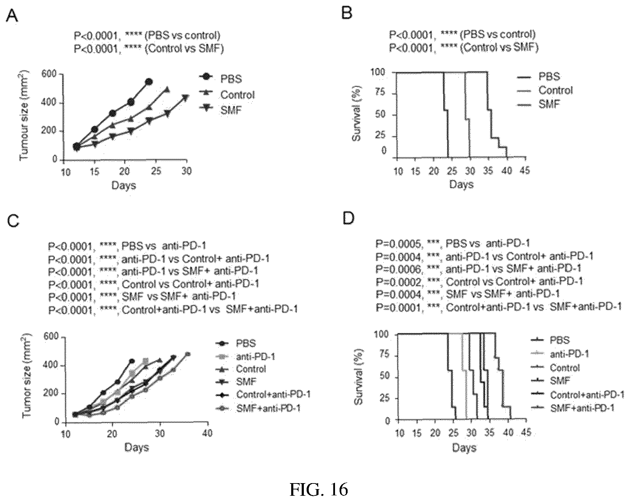

Fig. 16 shows CTLs treated by transplantation of static magnetic field for tumor immunotherapy. Melanoma inoculated mice were injected with PBS, control CTLs, and static magnetic field treated CTLs cells, respectively. Tumor size (A; n = 9) and survival (B; n = 9) were monitored. (C, D) CTLs in combination with anti-PD-1 antibody were tested for antitumor efficacy, including control CTLs + anti-PD-1, static magnetic field treated CTLs + anti-PD-1, anti-PD-1, and PBS, while control CTLs as well as magnetic field treated CTLs was compared (n = 7).

### Embodiments

The inventor studies the effects of a moderate static magnetic field on the behavior of immune cell cells using mice as a research model. The results demonstrate that the moderate static magnetic field is able to not only promote the secretion of granzymes and cytokines from immune cells (e.g., CD8⁺ T) cells, but also increase the levels of ATP and mitochondrial respiration. Specifically, the inventor finds that the moderate static magnetic field promotes the secretion of granzymes and cytokines, as well as the levels of ATP and mitochondrial respiration, from immune cells (e.g., CD8⁺ T) by regulating the expression of mitochondrial respiratory chain genes. In the case of knockdown of the mitochondrial respiratory chain genes *Uqcrb* and *Ndufs6,* the rise in granzyme and cytokine secretion by immune cells (e.g. CD8 + T) by the magnetic field treatment as well as the rise in the expression of ATP were able to be inhibited. The inventor also finds that the magnetic receptor candidate genes *Iscal* and *Cryl*/*Cry2* are involved in the regulation of mitochondrial respiratory chain genes *Uqcrb* and/or *Ndufs6.* Knockdown of Isca1 or Cry1/Cry2 results in downregulation of Uqcrb and/or Ndufs6 expression levels, upregulation of granzyme and cytokine secretion by immune cells (e.g., CD8⁺ T) in response to magnetic field treatment, and upregulation of ATP expression. The inventor demonstrates *in vivo* that a moderate static magnetic field can enhance the antitumor effect of immune cells (e.g., CD8⁺ T) and that reinfusion of magnetic field treated CTLs cells *in vivo* has a significant antitumor effect.

The magnetic field described herein can be a static magnetic field (stable magnetic field) with a moderate strength. The magnetic field strength of a moderate static magnetic field may be 1 mT-1.0 T, 0.05-0.9 T, 0.1-0.8 T, 0.2-0.7 T, 0.3-0.6 T, 0.4-0.5 T, for example, 0.3 T and 0.6 T, preferably 0.3 T. The magnetic field can be provided by permanent magnets or electromagnets. In the *in vitro* experiment herein, the 0.3 T permanent magnet was shown in Fig.1A, and the 0.6 T permanent magnet was shown in Fig.lB. Typically, the surface magnetic field strength of a permanent magnet will weaken with the pulling of the measured distance, and decay by 5 mT for approximately every 1 mm increase in the magnetic field strength. In one aspect, the invention provides a magnetic plate for *in vivo* experiments in mice, which is a magnet loaded resin fiber plate onto which a button magnet of 0.3 T or 0.6 T is set or embedded. Exemplarily, each button magnet has a diameter of 1 cm and each two mosaic magnets are separated by 2.5 cm. The surface magnetic field strength of the button magnet weakens with the pulling of the measured distance, decay by 50 mT for approximately every 1 mm increase in magnetic field strength. In another aspect, the invention provides a mouse cage comprising said magnetic plate. The size of the magnetic plate is designed according to the size of the mouse cage. When used, the magnetic plate could be placed on the bottom of the mouse cage (as shown in Fig. 1C). In examples, the permanent magnet and magnetic plate are placed with the N-pole facing up and the S-pole facing down. The method of preparing the device described above involves placing the body carrying the magnet described herein at the top, bottom, side, central position of the mammalian cage, preferably at the bottom.

The inventor finds that treatment of cells with a moderate static magnetic field induces upregulation of the expression or activity of the mitochondrial respiratory chain related genes *Uqcrb* and/or *Ndufs6* of the cells. In one or more embodiments, the expression or activity of *Uqcrb* is upregulated by at least 20%, at least 30%, at least 40%, at least 50%, at least 60%, at least 70%, or at least 90%. In one or more embodiments, the expression or activity of Ndufs6 is upregulated by at least 20%, at least 30%, at least 40%, at least 50%, at least 60%, at least 70%, or at least 90%. The treatments described are placing the cells in a magnetic field for at least 48 h, at least 60 h, at least 72 h, at least 84 h, at least 96 h, or at least 108 h. The inventor also finds that upregulated expression or activity of the mitochondrial respiratory chain related genes Uqcrb and/or Ndufs6 are regulated by the magnetic receptor related genes *Iscal, Cry1* and/or *Cry2.* Therefore, respiration of the cell is enhanced, and ATP level is increased. Based on this, after killing T cells by the moderate static magnetic field treatment, the level of mitochondrial respiration can be increased to provide energy for killing T cells and thus improve the killing efficiency. In addition, the inventor finds that the magnetic field herein enhances the expression or secretion of perforin, granzymes, and/or cytokines by upregulating the expression or activity of mitochondrial respiratory chain related genes (e.g., *Uqcrb* and/or *Ndufs6*) of T cells, resulting in enhanced T cell killing activity. Therefore, treatment of T cells with a moderate static magnetic field can be used to promote the expression or secretion of perforin, granzymes, and/or cytokines, which is used to increase the cell killing activity of the cells. In addition, after the treatment of bioreactor cells with a moderate static magnetic field, the level of mitochondrial respiration of the bioreactor cells can be increased, which in turn increases the reaction efficiency and yield.

The cells of the present invention may be any cells which benefit from an enhanced level of respiration, such as cells of a bioreactor or immune cells. Immune cells include lymphocytes, dendritic cells, monocytes/macrophages, granulocytes, and mast cells. When referring to immune cells, "activated" used herein refers to improved killing activity or killing ability of immune cells, so "activated immune cells" or "activated T cells" are immune cells with improved killing activity. In one or more embodiments, the immune cells are T cells. The T cells of the invention may be any kind of T cells including helper T cells, suppressor T cells, effector T cells, cytotoxic T cells, naive or native T cells, and memory T cells. Exemplary T cells are CD8⁺ T cells or CD4⁺ T cells. T cells can also be cells engineered for therapy, such as chimeric antigen receptor T cells (CAR-T). Therefore, the present method of magnetic field treatment of cells can be combined with chimeric antigen receptor technology. To upregulate the expression or activity of the respiratory chain related genes Uqcrb and/or Ndufs6 by T cells, T cells can be subjected to magnetic field treatment or the expression or activity of the respiratory chain related genes *Uqcrb* and/or *Ndufs6* can be elevated by methods well known in the art. Upregulation of the above genes upregulation of their expression can be achieved by overexpression in the host or host cells. Therefore, the agents that upregulates the expression of the above genes are the expression vectors of the genes. For example, conventional techniques in the art can be utilized to construct expression vectors suitable to express Isca1, Cry1/Cry2, Uqcrb, and/or Ndufs6 in host cells and transferred into host cells by conventional methods such that the expression vector expresses the molecule in the host cell, thereby achieving upregulation of its expression. The expression vector comprises additional components required for the expression of these genes in the host cell, and one skilled in the art is aware of these components. In certain embodiments, the expression of genes upstream of such molecules may be regulated, thereby elevating the expression of such molecules. For example, in certain embodiments, viral vectors (e.g., lentiviral vectors) expressing Isca1, Cry1/Cry2, Uqcrb, and/or Ndufs6 may be administered to the subject, thereby increasing the level of gene expression in the subject's cells. Expression vectors containing the coding sequences of Isca1, Cry1/Cry2, Uqcrb, and/or Ndufs6 are available to those skilled in the art based on the gene or amino acid sequences published on NCBI. T cells treated with magnetic fields or overexpressing the above genes have increased expression or secretion of perforin, granzymes, and/or cytokines, higher ATP level, and enhanced cell killing.

The cytokines described herein comprise one or more of those selected from the group consisting of: interleukin (IL), colony-stimulating factor (CSF), interferon (IFN), tumor necrosis factor (TNF), transforming growth factor-β Family (TGF-β), growth factors (GFS), and chemokine family. Specifically, the cytokines comprise one or more of those selected from the group consisting of: IL-1 ~ IL-38, G-CSF, M-CSF, GM-CSF, multi-CSF (IL-3), SCF, EPO, IFN-α, IFN-β and IFN-γ, TNF-α and TNF-β, TGF-β1. TGF-β2, TGF-β3, TGFβ1β2 as well as bone morphogenetic protein (BMP), epidermal growth factor (EGF), platelet-derived growth factor (PDGF), fibroblast growth factor (FGF), hepatocyte growth factor (HGF), insulin-like growth factor-I (IGF-I), IGF-II, leukemia inhibitory factor (LIF), nerve growth factor (NGF), oncostatin M (OSM), platelet-derived endothelial cell growth factor (PDECGF), transforming growth factor-α (TGF-α), VEGF, melanoma cell growth stimulating activity (GRO/MGSA), platelet factor-4 (PF-4), platelet basic protein, inflammatory protein 10 (IP-10), ENA-78, macrophage inflammatory protein 1α (MIP-1α), MIP-1β, RANTES, monocyte chemoattractant protein-1 (MCP-1/MCAF), MCP-2, MCP-3 and I-309, lymphocyte chemoattractant protein.

As used herein, magnetic field treated T cells, an agent that upregulates mitochondrial respiratory chain related genes (e.g., *Uqcrb* and/or *Ndufs6*) can be used to treat diseases, particularly those targeted by CD8⁺ T cells. Therefore, the diseases described herein encompass various types of cancer associated with CD8⁺ T cells, including solid tumors and hematologic neoplasms such as adenocarcinomas, lung, colon, colorectal, breast, ovarian, cervical, gastric, cholangiocarcinomas, gallbladder, esophageal, pancreatic, and prostate cancers, as well as leukemia and lymphomas such as B cell lymphomas, mantle cell lymphomas, acute lymphoblastic leukemias, chronic lymphocytic leukemia, hairy cell leukemia, and acute myeloid leukemia, among others. Preferably, the cancer is melanoma and/or breast cancer.

In another aspect, the invention provides a pharmaceutical composition comprising the T cells described herein and a pharmaceutically acceptable excipient. "Pharmaceutically acceptable excipient" used herein means vehicles, diluents and/or excipients that are pharmacologically and/or physiologically compatible with the subject and the active ingredient, including, but not limited to: pH modifying agents, surfactants, carbohydrates, adjuvants, antioxidants, chelators, ionic strength enhancers, preservatives, carriers, fluidizers, sweeteners, dyes/colorants, odorants, wetting agents, dispersants, suspending agents Stabilizers, isotonic agents, solvents or emulsifiers. In some embodiments, the pharmaceutically acceptable excipient may include one or more inactive ingredients including, but not limited to: stabilizing agents, preservatives, additives, adjuvants, sprays, compressed air or other suitable gases, or other suitable inactive ingredients for use with a pharmacodynamic compound. More specifically, suitable pharmaceutically acceptable excipients may be those commonly used in the art for plant extraction of ingredients or nucleic acid administration.

Typically, a pharmaceutical composition contains a therapeutically effective amount of magnetic field treated T cells described herein, T cells in which mitochondrial respiratory chain related genes (e.g., *Uqcrb* and/or *Ndufs6*) are upregulated, or agents that upregulate mitochondrial respiratory chain related genes (e.g., *Uqcrb* and/or *Ndufs6*). A therapeutically effective amount refers to a dose at which the treatment, prevention, mitigation, and/or mitigation of a disease or condition can be achieved in a subject. The effective amount of treatment can be determined based on the patient's age, sex, the condition being affected and its severity, and the patient's other physical condition. A therapeutically effective amount may be administered as a single dose or may be administered in multiple doses depending on the effective treatment regimen. Herein, a subject or patient usually refers to a mammal, especially a human.

T cells of the present invention can be administered alone or as a pharmaceutical composition. The cellular or pharmaceutical compositions of the invention may be administered in a manner suitable for the treatment (or prevention) of a disease. The number and frequency of administration will be determined by various factors, such as the patient's condition, and the type and severity of the patient's disease. Administration of the compositions may be carried out in any convenient manner, including by injection, infusion, implantation, or transplantation. The compositions described herein may be administered to a patient subcutaneously, intradermally, intratumorally, intranodally, intraspinally, intramuscularly, by intravenous injection, or intraperitoneally. In one embodiment, the T cell composition of the invention is administered to a patient by intradermal or subcutaneous injection. In another embodiment, the T cell composition of the invention is preferably administered by intravenous injection. Compositions of T cells can be injected directly into tumors, lymph nodes, or sites of infection.

The T cells or agents described herein may be used in combination with other agents that elevate the mitochondrial respiration of the cell, agents that elevate the cellular ATP level, agents that promote the expression or secretion of perforin, granzymes, and/or cytokines of the cell, agents that improve the cell killing capacity of the cell, or agents that treat cancer, such as anti-PD-1 antibodies. Those skilled in the art may determine the administered dose of other agents. T cells or reagents of the present invention may also be combined with other therapies known in the art. The therapies described include, but are not limited to, chemotherapy, radiotherapy, and immunosuppressive agents. For example, treatments may be carried out in combination with radiotherapy or chemotherapy formulations that are well known in the art to treat tumor antigen mediated diseases.

The invention further comprises a method of increasing the ATP level of a cell, promoting mitochondrial respiration of the cell, promoting the expression and/or secretion of perforin, granzyme and/or cytokine by the cell, and enhancing the killing capacity of T cells, comprising one or more steps selected from the group consisting of: (1) treating the cell using a magnetic field described herein or a magnetic field produced by the device herein, (2) upregulating the expression and/or activity of mitochondrial respiratory chain related genes of the cell, such as *Uqcrb* and/or *Ndufs6.*

In another aspect, the invention provides methods of increasing the ATP level of a subject's cells, promoting mitochondrial respiration of the subject's cells, promoting the expression and/or secretion of perforin, granzyme and/or cytokine by the subject's cells, enhancing the subject's T cell killing ability, or treating the subject's cancer. The method comprises administering to a subject in need a therapeutically effective amount of the T cells, agents, or pharmaceutical compositions described herein. Alternatively, or additionally, the method comprises treating the subject using a magnetic field described herein or a magnetic field generated by the device herein.

"Anti-tumor effect" as used herein refers to a biological effect that can be represented by a decrease in tumor volume, a decrease in the number of tumor cells, a decrease in the number of metastases, an increase in life expectancy, or an improvement in various physiological symptoms associated with cancer.

The present invention stimulates CD8⁺ T cells *in vitro* with anti-CD3/anti-CD28 antibodies for 96 h and simultaneously treats the CD8⁺ T cells with a 0.3 T magnet to obtain CTL effector cells. This CTL effector cells demonstrate significant antitumor efficacy *in vitro* and *in vivo.* The CD8⁺ T cells obtained by treating CD8⁺ T cells with a static magnetic field of 0.3 T-0.6 T with TCR stimulation for 72 h exhibite significantly increased secretion of granzymes and cytokines and enhanced antitumor capacity. Treatment of mice with a 0.6 T magnetic plate to form a magnetic field with a surface magnetic average of 0.31 T significantly inhibites tumor growth in mice.

The invention is further described in detail by reference to the following experimental examples. These examples are provided for illustrative purposes only and are not intended to be limiting unless otherwise specified. Thus, the present invention should by no means be construed as being limited to the following embodiments, but rather should be construed as including all the variations that become apparent as a result of the teaching provided herein. The methods and reagents used in the examples, unless otherwise stated, are those conventional in the art.

### Example

### I. Experimental materials and methods

### 1. Mice

C57BL/6 wild-type mice were purchased from SLAC, and OT-I TCR transgenic mice as well as PyMT mice were obtained from Jackson Laboratories, USA. All experimental mice were housed in an SPF grade animal house at Shanghai Institutes for Biological Sciences, Chinese Academy of Sciences. All animal experiments were approved by the institutional animal care committee of the Institute of Biochemistry and Cell Biology, Chinese Academy of Sciences.

### 2. Cell lines

B16-F10-OVA and EL-4 cells were kindly provided by the researcher Xu Chenqi, Institute of Biochemistry and cell biology, Shanghai Institutes for Biological Sciences, Chinese Academy of Sciences. B 16-F10 cells were cultured in DMED medium supplemented with the following: 10% serum, L-glutamine (2 mM), penicillin (100 U/ml), and streptomycin (10 U/ml). EL-4 cells were cultured in RPMI 1640 medium supplemented with the following: 10% serum, L-glutamine (2 mM), penicillin (100 U/ml), streptomycin (10 U/ml).

### 3. Permanent magnet and magnetic plate

Magnets with surface magnetic field strengths of 0.3 T and 0.6 T were purchased from Hangzhou Permanent Magnet Group Co., LTD. The size of the 0.3 T permanent magnet was 10 cm long × 10 cm wide × 5 cm high (Fig. 1A), and the size of the 0.6 T permanent magnet was 10 cm long × 10 cm wide × 10 cm high (Fig. 1B). The surface magnetic field strength of the permanent magnet will weaken with the pulling of the measured distance, decay by 5 mT for approximately every 1 mm elevation in the magnetic field strength. Magnetic plates for *in vivo* experiments in mice were designed and provided by Zhejiang HeYe Health Technology Co., Ltd. and were embedded with button magnets at 0.3 T and 0.6 T, respectively, in resin fiber plates. Each button magnet had a diameter of 1 cm, and each two button magnets were separated by 2.5 cm. The surface magnetic field strength of the button magnet weakens with the pulling of measured distance, decay by 50 mT for approximately every 1 mm elevation in the magnetic field strength. The size of the magnetic plate was designed according to the size of the mouse cage, which was 32 cm long × 12.5 cm wide × 1.5 cm high, and was placed on the bottom of the mouse cage when used (Fig. 1C). All permanent magnets and magnetic plates in this study were placed with the magnet N-pole facing up and the S-pole facing down.

### 4. Antibodies

The following antibodies were all of murine origin: anti-CD4 (RM4-5), anti-CD8a (53-6.7), anti-CD44 (IM-7), anti-CD621 (MEL-14), anti-CD69 (H1.2F3), anti-Ly6G (Gr1) (RB6-8C5), anti-CD11b (M1/70), anti-TNFα (MP6-XT22), anti-IL-2 (JES6-5H4), and anti-IL-4 were purchased from BD. Anti-mFoxp3 (FJK-16s), anti-granzyme B (NGZB) and anti-IL-17 (eBio17B7) were purchased from eBioscience. Anti-CD25 (PC61), anti-IFNy (XMG1.2) and FITC Annexin V were purchased from Biolegend. Anti-CD3 (145-2C11) and anti-CD28 (37.51) antibodies for T cell *in vitro* stimulation experiments were purchased from BD.

### 5. T cell isolation and staining

CD4⁺ T cells or CD8⁺ T cells were obtained from spleens of C57BL/6 mice, purified by CD4⁺ T cell or CD8⁺ T cell negative selection kits (Stem Cell), and then cultured and stimulated in 48 well plates coated with 1 µg ml⁻¹ of anti-CD3 and anti-CD28 antibodies. On the first day of stimulation, cells were seeded at a density of 0.7 × 10⁶ per well and cell passage was performed at 1:2 ratio at 48 h of stimulation. Magnet test group cells in 48 well plate were placed on permanent magnet for culture, and control cells without magnet treatment were cultured normally. For isolation of tumor infiltrating lymphocytes, mouse tumor was lysed and then digested with collagenase IV before gradient centrifugation with 40 - 80% (V/V) Percoll (GE Corporation) to obtain tumor infiltrating lymphocytes. When testing cytokine secretion by CD4⁺ T cells or CD8⁺ T cells, purified cells were required to be restimulated with PMA (50 ng ml⁻¹) and ionomycin (1 µM) and 5.0 µg ml⁻¹ brefeldin A for 4 h before staining. For intracellular staining, cells were fixed with 1 ml of 2% formaldehyde in PBS for 10 min at room temperature and stained after punching the wells with intracellular staining buffer for 5 min. TCR unstimulated T cells or samples stained with isotype antibodies served as negative controls. Cell fluorescence was examined by a FACSCalibur (BD Biosciences) flow cytometer, and the data obtained were analyzed with FlowJo software.

### 6. CD8⁺ T cell in vitro killing assay

CD8⁺ T cell *in vitro* killing assays were performed according to the experimental methods of Qiu Xiaoyan research team (2014). Spleens from OT-I transgenic mice were removed and CD8⁺ T cells were purified using a negative kit, and then stimulated *in vitro* with anti-CD3/anti-CD28 antibodies for 96 h as CTL effector cells and simultaneously treated with a 0.3 T magnet, whereas controls were not treated with a magnet. EL-4 cells were used as target cells which were subjected to killing assays after 30 min incubation at 37 °C with 10 nM OVA₂₅₇₋₂₀₄. CTLs and EL-4 cells were resuspended with killing buffer (phenol red free RPMI 1640 with 2% serum) after washing three times with PBS, and the two kinds of cells were diluted into gradient concentrations of 1.25:1, 2.5:1, and 5:1, respectively. The two kinds of cells were incubated at 37 °C for 4 h, and the killing efficiency was indicated by the detection of lactate dehydrogenase (LDH) level using the CytoTox 96 killing Kit (Promega).

### 7. CD8⁺ T cell in vivo killing assay

CD8⁺ T cell *in vitro* killing assay was performed according to to the experimental method of Wang Hongyan research team (2015). CTL cells were similarly obtained from OT-I transgenic mice as in the above experiments and treated with a 0.3 T magnet, and control cells were not treated with a magnet. CTLs (3 × 10⁶) were injected into C57BL/6 recipient mice via tail vein, and 4 mice were followed by tail vein injection of CFSE labeled splenocytes (3 × 10⁶): CFSE^{low}-labeled splenocytes incubated with 10 nM OVA2₅₇₋₂₆₄ (1 µM) were mixed 1:1 with CFSE^{high}-labeled splenocytes that had not been incubated with OVA2₅₇₋₂₆₄ (10 µM). After 6 h, the spleens of recipient mice were taken and flow analyzed for the expression of CFSE to detect the killing efficiency, killing efficiency = [1-(% CFSE^{low})/(% CFSE^{high})] × 100%.

### 8. Melanoma mouse model suppresses static magnetic field treated CTLs

T cell transplantation experiments were performed according to the experimental methods of the Xu Chenqi research team (2016). 8-10 weeks old C57BL/6 mice were inoculated subcutaneously with B16F10-OVA cells in groin, and each mouse was injected with 2 × 10⁵ cells. On day 12 of tumor inoculation, mice with excessively large or small tumors were removed to exclude individual differences, and mice with consistent tumor sizes were divided into three groups with 7 - 10 mice in each group, they received tail vein injections of CTLs treated with a 0.3T magnet (1.5 ×10⁶), control CTLs (1.5 × 10⁶) as well as PBS. In which the preparation of CTLs is described in section "6". When testing the combination of CTLs with anti-PD-1 antibody, the following three groups of mice were simultaneously compared for tumor growth and survival: (1) injection with anti-PD-1 antibody alone; (2) CTLs treated with injection of anti-PD-1 antibody and 0.3 T magnet simultaneously; (3) injection with both anti-PD-1 antibody and control CTLs. Anti-PD-1 antibody (RMP1-14, Bio X Cell, 200 µg per mouse per injection), injected intraperitoneally every three days for a total of three consecutive injections. Since the mice were inoculated with B16 cells on day 12, the tumor size was measured every two days, and the survival of the mice was recorded daily. Tumor size was measured with longth × width value, and death was determined with a tumor diameter of more than 20 mm in accordance with mouse ethical welfare.

### 9. Tumor growth and histochemical detection in PyMT breast cancer mice

PyMT transgenic mice (FYB background) were treated with 0.3 T or 0.6 T magnetic plates at day 21, whereas control mice were treated with resin fiber plates without a magnet. The mice were observed for developing tumors every 2 days, and the tumor size of the mice was measured weekly with vernier calipers. Tumor size was expressed by a numerical value of A×B²/2: "A" represents for the longitudinal length (mm); "B" represents for the transverse length (mm). When tumors grew to 10-15 mm, tumor tissue masses were taken and embedded in paraffin and sectioned after fixation with 4% formaldehyde overnight, followed by HE staining. HE staining results were detected with an Olympus BX51 microscope and photographed with DP71 camera (Olympus).

### 10. RNA sequencing and bioinformatics analysis

The RNA concentration, purity, and integrity of the extracted RNA were analyzed using an Agilent Bioanalyzer 2100 (Agilent Technologies, santaclara, CA, US). RNA libraries were established using SMARTer Stranded TotalRNA-Seq Kit-Pico Input Mammalian (Illumina) and the libraries were sequenced with IlluminaHiSeq X-Ten sequencer. After sequencing, Trimmomatic (version 0.36) was used for quality filtering and to remove sequencing adapters, RNA SEQ reads were aligned to the mouse genome via HISAT2 and only reads that matched a single genomic position were used to calculate gene expression using StringTie. All gene expressions were normalized to FPKM, fold change of gene expression as well as p-value were estimated using Balltown. Only genes with fold change ≥ 2 and p-value ≤ 0.05 were identified as differentially expressed genes. GO DAVID (modified Fisher exact p-value < 0.05) was calculated. All sequencing results in this study have been displayed on Gene Expression Omnibus (GEO) of NCBI under the accession number GSE113858 (link: https://www.ncbi.nlm.nih.gov/geo/query/acc.cgi?acc=GSE113858, token: orqlmoqkfbwpxgp).

### 11. RNA extraction, reverse transcription and real-time quantitative PCR

Total RNA from cell samples was extracted with Trizol (Invitrogen) and subsequently reverse transcribed with the Superscript III First-Strand Kit (Invitrogen), followed by real-time quantitative PCR (RT-PCR).

The primers of RT-PCR are as follows:
*Actb* (forward, GACGGCCAGGTCATCACTATTG (SEQ ID NO:1); reverse, AGGAAGGCTGGAAAAGAGCC (SEQ ID NO:2));
*Ndufc1* (forward, CACGGTCGAAGTTCTATGTC (SEQ ID NO:3); reverse, TTGTGTGTTTGGATGAGATAAATC (SEQ ID NO:4));
*Atp6v0b* (forward, AGTTGCTCTACCTCGGGATCT (SEQ ID NO:5); reverse, ATGCCACATCAAAGCGAAAGC (SEQ ID NO:6));
*Idh3b* (forward, AGGCACAAGATGTGAGGGTG (SEQ ID NO:7); reverse, CAGCAGCCTTGAACACTTCC (SEQ ID NO:8));
*Ndufs6* (forward, GGGGAAAAGATCACGCATACC (SEQ ID NO:9); reverse, CAAAACGAACCCTCCTGTAGTC (SEQ ID NO:10));
*mt-Nd4* (forward, GAGTTCACCTATGACTACCA (SEQ ID NO:11); reverse, CTAGAATAATGGAGATGCGAAT (SEQ ID NO:12));
*Uqcrc2* (forward, AAAGTTGCCCCGAAGGTTAAA (SEQ ID NO:13); reverse, GAGCATAGTTTTCCAGAGAAGCA (SEQ ID NO:14));
*Uqcrb* (forward, GGCCGATCTGCTGTTTCAG (SEQ ID NO:15); reverse, CATCTCGCATTAACCCCAGTT (SEQ ID NO:16));
*Ndufa13* (forward, ACGGCCCCATCGACTACAA (SEQ ID NO:17); reverse, CCTGGTTCCACCTCATCATTCT (SEQ ID NO:18));
*Atp5k* (forward, GTTCAGGTCTCTCCACTCATCA (SEQ ID NO:19); reverse, CGGGGTTTTAGGTAACTGTAGC (SEQ ID NO:20));
*Uqcrfs1* (forward, GAGCCACCTGTTCTGGATGTG (SEQ ID NO:21); reverse, GCACGACGATAGTCAGAGAAGTC (SEQ ID NO:22));
*Cox7b* (forward, TTGCCCTTAGCCAAAAACGC (SEQ ID NO:23); reverse, TCATGGAAACTAGGTGCCCTC (SEQ ID NO:24));
*Ndufa10* (forward, ACCTTTCACTACCTGCGGATG (SEQ ID NO:25); reverse, GTACCCAGGGGCATACTTGC (SEQ ID NO:26));
*mt-Nd5* (forward, CCAACAACAACGACAATCTA (SEQ ID NO:27); reverse, TTGAGTGTAGTAGTGCTGAA (SEQ ID NO:28));
*mt-Nd3* (forward, CTCTTCTACTTCCACTACCAT (SEQ ID NO:29); reverse, GCCTAGAGATAGAATTGTGACTA (SEQ ID NO:30));
*mt-Nd4l* (forward, CTTCTTCAACCTCACCATAG (SEQ ID NO:31); reverse, CTTCCAGGCATAGTAATGTG (SEQ ID NO:32));
*mt-Atp8* (forward, GGTAATGAATGAGGCAAATAGA (SEQ ID NO:33); reverse, GTAATGAATGAGGCAAATAGATT (SEQ ID NO:34));
*Gzmb* (forward, TCTCGACCCTACATGGCCTTA (SEQ ID NO:35); reverse, TCCTGTTCTTTGATGTTGTGGG (SEQ ID NO:36));
*Tnf* (forward, CTGGATGTCAATCAACAATGGGA (SEQ ID NO:37); reverse, ACTAGGGTGTGAGTGTTTTCTGT (SEQ ID NO:38));
*Ifng* (forward, TCCTCGCCAGACTCGTTTTC (SEQ ID NO:39); reverse, GTCTTGGGTCATTGCTGGAAG (SEQ ID NO:40));
*Iscal* (forward, CTTAAAGACAAACCTGAGCAT (SEQ ID NO:41); reverse, TTCCACATAGTCCATCTCTG (SEQ ID NO:42));
*Cry1* (forward, TTGAAGAGTTACTGCTTGATG (SEQ ID NO:43); reverse, ACGCCTAATATAGTCTCCATT (SEQ ID NO:44));
*Cry2* (forward, TGTGGGCATCAACCGATGG (SEQ ID NO:45); reverse, CGGACTACAAACAGACGCGAA (SEQ ID NO:46)).

### 12. Primary T cell gene knockdown experiments

The constructed MLP shRNA plasmids were transfected into Plat-E cells using the calcium phosphate method, and viral supernatants were collected 48 h later. After mixing the virus solution and RPMI-1640 complete medium at a ratio of 1:1, which was used to stimulate the T cells infected with anti-CD3 (1 µg/ml) and anti-CD28 (1 µg/ml) for 24 h, ploybrene was added to a final concentration of 4 µg/ml. It was centrifuged at 1100g for 2 h at 30°C in a horizontal centrifuge. After being placed in a 37°C incubator for 10 h, the viral fluid was replaced with fresh RPMI-1640 complete medium. Cells were stimulated with anti-CD3 (1 µg/ml) and anti-CD28 (1 µg/ml) for another 72 h and treated with a 0.3T magnet, while control cells were not treated with a magnet. Cells positive for GFP were sorted by flow analyzer, RNAs were extracted and reverse transcribed followed by RT-PCR to measure gene knockdown efficiency. The shRNA primer sequences constructed were as follows:
*Uqcrb*-target shRNA: 
5'-TGCTGTTGACAGTGAGCGCAAGAAGTGATCTTTTAGTTAATAGTGAA
GCCACAGATGTATTAACTAAAAGATCACTTCTTTTGCCTACTGCCTCGGA
-3'. (SEQ ID NO:47)
*Ndufs6*-target shRNA: 
5'-TGCTGTTGACAGTGAGCGCGCCATTGATTTGATAGCACAATAGTGAA
GCCACAGATGTATTGTGCTATCAAATCAATGGCATGCCTACTGCCTCGG
 A-3'. (SEQ ID NO:48)
*Isca1*-target shRNA: 
5'-TGCTGTTGACAGTGAGCGATGCCTCGTGGGTGAAAATAAATAGTGAA
GCCACAGATGTATTTATTTTCACCCACGAGGCAGTGCCTACTGCCTCGG
A-3'. (SEQ ID NO:49)
*Cry1*-target shRNA: 
5’-TGCTGTTGACAGTGAGCGCCCGCCTCTTTATTTACATCTATAGTG
AAGCCACAGATGTATAGATGTAAATAAAGAGGCGGATGCCTACTGCCTC
GGA-3’. (SEQ ID NO:50)
Cry2-target shRNA:

5'-TGCTGTTGACAGTGAGCGCCAGTTTGTTTGTGAATATTTATAGTG
AAGCCACAGATGTATAAATATTCACAAACAAACTGTTGCCTACTGCCTC
GGA-3'. (SEQ ID NO:51)

### 13. Determination of cellular ATP concentration

For each test sample, 10⁵ CD8⁺ T cells were taken for detecting ATP concentration using an ATP Test Kit (S0027, Beyotime) according to the manufacturer's instructions, and chemiluminescence values of samples were measured using GloMax^{®} 20/20 chemiluminescence detector.

### 14. Mitochondrial respiration test

OCRs (oxygen consumption rate) and ECARs (extracellular acid producing capacity) of CD8⁺ T cells were measured using Seahorse MitoStress Test Kit with the following inhibitors added for detection: 1 µM oligomycin (to block ATP synthesis), 1 µM FCCP (uncoupler of mitochondrial oxidative phosphorylation), 1 µM rotenone and antimycin A (respiratory chain inhibitors), 10 mM glucose (induces glycolysis) and 100 mM 2-Deoxy-D-glucose (2-DG, glycolysis inhibitor). All test samples were tested with XF-24 Extracellular Flux Analyzer, Seahorse Bioscience.

### 15. Biostatistical Analysis

Statistical analysis was performed with Graphpad Prism 6 software. All statistics are presented as mean ± standard error of the mean (mean ± S.E.M.). Tumor free curves were analyzed using the logrank test method. Comparisons between two independent experimental groups were performed using a two tailed t-test, and P values less than 0.05 were considered significant. *P < 0.05; **P < 0.001; ***P < 0.0001, ****P < 0.0001.

### II. Experimental results

### 1. Moderate magnetic field promotes granzyme and cytokine secretion by CD8⁺ T cells

1.1 To identify the effects of moderate static magnetic fields on T cell function, we first performed *in vitro* TCR stimulation of T cells and compared their activation under conditions of different stimulation times and treatment with different magnetic field strengths. We purified CD4⁺ T cells and CD8⁺ T cells from splenic lymphocytes of C57/B6 mice and then cultured them in 48 well plates. The magnetic field treatment group placed the 48 well plates on a permanent magnet with a magnetic field strength of 0.3 T or 0.6 T (Fig. 1, A, B), whereas the control group was not treated with a magnet. We detected the expression of these activating molecules, CD69, CD44, and CD25, in response to TCR stimulation for 24 h, 48 h, and 72 h. The results showed that for CD4⁺ T cells, the expression of CD44 of 0.3 T magnetic field treated cells was slightly higher than that of the control group at all time points, while no significant difference was observed at 0.6 T, nor were CD69 and CD25. Whereas for CD8⁺ T cells, the expression of CD25 of 0.3 T-treated cells was slightly higher than that of the control at 48 h and 72 h, but not for 0.6 T, nor did CD69 and CD44 (Fig. 2).

1.2 Next, we examined the effect of moderate magnetic fields on cytokine secretion by CD4⁺ T cells and CD8⁺ T cells. The results showed that the moderate magnetic field did not significantly affect cytokine secretion by CD4⁺ T cells at all time points examined (Fig. 3).

1.3 Interestingly, treatment with magnetic fields at 0.3 T and 0.6 T significantly enhanced the secretion of granzymes and IFNγ, NFα, cytokines from CD8⁺ T cells under conditions of TCR stimulation for 72 h (Fig. 4, A, B), whereas no significant differences were observed between conditions of TCR stimulation for 24 h and 48 h (Fig. 4, C, D). To exclude the interference of nonmagnetic impurities in the magnet, we also compared the effects of treatment with metallic materials without magnetism (the same size as the 0.3 T magnet) (Fig. 5, "pseudotyped" group) and no magnet treatment (Fig. 5, "control" group) as well as treatment with a 0.3 T magnet (Fig. 5, "0.3 T" group) on CD8⁺ T cell cytokine secretion. The results showed that granzyme and cytokine secretion of CD8⁺ T cell were not significantly different between the "control" and "pseudotyped" groups, whereas that of 0.3 T magnet treatment was significantly elevated compared with either the "control" or "pseudotyped" group (Fig. 5, A, B). This illustrated that the 0.3 T magnet treatment promotes granzyme and cytokine secretion of CD8⁺ T cells, whereas the nonmagnetic impurities did not have this biological effect. Subsequently, we examined the effect of the magnetic field on the transcription level of granzyme and cytokine genes of CD8⁺ T cells. The results showed that the expressions of *Gmzb* and *Ifnr* genes were obviously elevated in CD8⁺ T treated with 0.3 T magnetic field, while *Tnf* showed no significant change (Fig. 4, E).

1.4 Next, we examined whether the static magnetic field affects the proliferation and survival of CD8⁺ T cells. We examined the effect of the magnetic field on CD8⁺ T cell proliferation using a CFSE labeling assay, which showed that treatment with a 0.3 T magnetic field did not affect cell proliferation (Fig 6, A). Moreover, magnetic field treatment did not affect apoptosis and death of CD8⁺ T cells (Fig. 6, B, C). The above results showed that a moderate static magnetic field promoted the secretion of granzymes and cytokines by CD8⁺ T cells, but not the proliferation and survival of CD8⁺ T cells, and did not affect cytokine secretion by CD4⁺ T cells.

### 2. Moderate static magnetic field upregulates respiratory chain gene expression

To investigate the molecular mechanisms by which a static magnetic field promotes granzyme and cytokine secretion by CD8⁺ T cells, we compared CD8⁺ T cells treated with a 0.3 T magnetic field (TCR stimulation for 72 h) with CD8⁺ T cells treated without a magnetic field (TCR stimulation for 72 h), as well as unstimulated CD8⁺ T cells, and performed a comparative RNA-Seq based transcriptome analysis (GEO: GSE113858). The main differentially expressed genes (fold change ≥ 2) could be separated into two clusters by functional enrichment analysis. Gene cluster 1 included 198 genes, mainly involved in mRNA metabolism and RNA processing (Fig. 7, A). Gene cluster 2 included 24 genes, mainly involved in ATP metabolism and mitochondrial respiratory chain transmission (Fig. 7, A, B). Gene cluster 2 attracted our interest because mitochondrial respiratory transport chain genes contained an abundance of iron sulfur protein genes, whereas studies have suggested that the iron sulfur protein genes were most likely magnetic receptor protein genes. Interestingly, most genes of gene cluster 2 were significantly upregulated in magnetic field treated cells compared with cells without magnetic field treatment (Figure 7, B).

Accordingly, we speculated that a magnetic field promoted granzyme and cytokine secretion by CD8⁺ T cell by upregulating the expression of mitochondrial respiratory chain related genes. Subsequently, we performed further validation of the upregulated expression of 16 respiratory chain related genes with real time PCR technology. The results showed that among these genes, *Uqcrb* and/or *Ndufs6* were significantly upregulated in magnetic field treated cells compared to control cells (Fig. 8, A). To further identify whether upregulation of Uqcrb or Ndufs6 is necessary for the secretion of granzymes and cytokines by CD8⁺ T cells, we applied a shRNA vector expression system to construct a knockdown system. We first evaluated the knockdown efficiency of *Uqcrb* and *Ndufs6* in primary CD8⁺ T cells and found that transfer of shRNA into these cells significantly downregulated Uqcrb or Ndufs6 expression (Fig. 8, B). Once *Uqcrb* or *Ndufs6* were effectively downregulated, the rise of both granzymes and cytokines in magnetically treated CD8⁺ T cells was significantly inhibited, and IFNγ in particular, turned from upregulated to downregulated (Fig. 8, C-H). These data suggest that static magnetic field most likely promotes granzyme and cytokine secretion by CD8⁺ T cell by upregulating the expression of mitochondrial respiratory chain related genes *Uqcrb* and *Ndufs6* genes.

### 3. Moderate static magnetic field enhances ATP production and mitochondrial respiratory capacity

The above results showed that the 0.3 T moderate static magnetic field upregulated the expression of ATP metabolism and mitochondrial respiratory chain related genes in CD8⁺ T cells under TCR stimulation for 72 h, suggesting that our static magnetic field may affect the ATP level and mitochondrial respiratory capacity of CD8⁺ T cells. We subsequently examined the effect of static magnetic field on ATP level of CD8⁺ T cells. The results showed that CD8⁺ T cells treated with a 0.3 T static magnetic field had a significantly higher ATP concentration than control cells (Fig. 9, A). We also detected mitochondrial oxygen consumption of CD8⁺ T cells to indicate mitochondrial respiratory capacity. The results showed that the OCR value (oxygen consumption rate) of CD8⁺ T cells treated with a static magnetic field was significantly higher than that of control cells (Fig. 9, B and D). In addition, the rate of ATP output as reflected by OCR was also significantly higher in CD8⁺ T cells treated with a static magnetic field than in control cells (Fig. 9, C, D), which is consistent with the results of the elevated ATP level. In addition, we examined the effect of a static magnetic field on the mitochondrial extracellular acid producing capacity (ECAR) of CD8⁺ T cells, and the results showed that the ECAR values of CD8⁺ T cells treated with a static magnetic field were lower than those of control cells (Fig. 9, E, F).

Our above results showed that a static magnetic field promoted the secretion of granzymes and cytokines by CD8⁺ T cells by upregulating the expression of mitochondrial respiratory chain related genes *Uqcrb* and/or *Ndufs6,* and we wondered whether the expression of these two genes was also involved in regulating the ATP level. We next did knockdown experiments for both genes, which showed that knockdown of either *Uqcrb* or *Ndufs6* significantly inhibited the phenomenon of elevated ATP level in magnetic field treated cells (Fig. 9, G). Taken together, these findings suggested that a static magnetic field boosted ATP level in CD8⁺ T cells most likely by upregulating the expression of the mitochondrial respiratory chain related genes *Uqcrb* and *Ndufs6.*

### 4. Magnetic receptor candidate genes involved in regulation of the expression of Uqcrb and Ndufs6

Since *Iscal* and *Cryl*/*Cry2* are well recognized as magnetic receptor candidate genes, we wondered whether a moderate static magnetic field regulated the expression of *Uqcrb* or *Ndufs6* through the magnetic receptor candidate genes. Our RNA-seq results indicated that genes with significant expression differences in CD8⁺ T cells treated with 0.3 T magnetic field compared to control cells did not include *Iscal* and *Cryl*/*Cry2* (Fig. 7, A, B). We subsequently validated this with RT-PCR and found no significant difference in expression levels of *Iscal* and *Cryl*/*Cry2* between 0.3 T magnetic field treated cells and control cells (Fig. 10, A), which was consistent with the RNA-seq results. Next, we used knockdown experiments of *Iscal* and *Cryl*/*Cry2* to examine their effects on Uqcrb and/or Ndufs6 transcription, and knockdown efficiency assays were shown in Fig. 10, B. The results showed that knockdown of *Iscal* or *Cryl*/*Cry2* was able to reverse the upregulation of *Uqcrb* and *Ndufs6* in magnetically treated cells, and the inhibitory effect was more pronounced without magnet treatment than with 0.3 T magnet treatment (Fig. 10, C, D). This could be due to the upregulation of Uqcrb and/or Ndufs6 by magnet treatment at 0.3 T (Fig. 8, A).

We next examined the effects of knocking down the magnetic receptor candidate genes on changes of granzymes and cytokines and ATP levels in CD8⁺ T cells as a result of magnetic field treatment. The results showed that in the presence of knockdown of Isca1 or Cry1/Cry2, the rise in both granzymes and cytokines was significantly inhibited in magnetically treated CD8⁺ T cells (Fig. 10, E-J). Furthermore, *Iscal* knockdown reversed the upregulation of gzmB and IFNγ in magnetically treated CD8⁺ T cells (Fig. 10, H-J). Furthermore, knockdown of *Iscal* or *Cryl*/*Cry2* equally inhibited the upregulation of ATP levels in CD8⁺ T cells in response to magnetic field treatment, and the effect for *Iscal* was more pronounced (Fig. 10, K). These data demonstrated that the magnetic receptor candidate genes *Iscal* and *Cryl*/*Cry2* are involved in regulating transcription levels of *Uqcrb* and/or *Ndufs6,* and that knockdown inhibits the upregulation of granzymes and cytokines and ATP levels in CD8⁺ T cells induced by 0.3 T magnetic field treatment.

### 5. Enhanced killing of CD8⁺ T cells by a moderate static magnetic field

CD8⁺ T cells, also known as killer cells (CTLs), kill tumor cells mainly by secreting perforin, granzymes, and cytokines. Since our above results indicated that a moderate static magnetic field enhances the secretion of granzymes and cytokines by CD8⁺ T cells, we subsequently examined the effects of a magnetic field on the killing capacity of CTLs. We isolated CD8⁺ T cells from spleens of OT-I TCR transgenic mice and performed *in vitro* TCR stimulation as effector cells, which are capable of specifically recognizing OVA₂₅₇₋₂₆₄; and EL-4 cells incubated with OVA2₅₇₋₂₆₄ were used as target cells. We examined CD8⁺ T killing activity by the LDH assay, which showed that CTLs cells treated with a moderate static magnetic field presented greater killing capacity than control cells (Fig. 11, A) and secreted higher levels of granzymes and cytokines (Fig. 11, B, C). This illustrated that a static magnetic field could enhance killing by enhancing granzyme and cytokine secretion of CD8⁺ T cells *in vitro,* so can this killing capacity be maintained after reinfusion into the body? We subsequently examined the killing activity *in vivo.* We injected static magnetic field treated CTLs and control CTLs into recipient mice via tail vein, and then mixed OVA₂₅₇₋₂₆₄-incubated spleen cells (CFSE^{low}) and no incubated spleen cells (CFSE^{high}) at a ratio of 1:1 as target cells for injection into recipient mice via tail vein. The experimental results indicated that the magnetic field treated CTLs exhibited stronger killing ability compared with the control cells (Fig. 11, D, E). The above data demonstrated that a static magnetic field could enhance the killing capacity of CD8⁺ T cells *in vitro* and maintain this effect *in vivo.*

### 6. Enhanced antitumor function of CD8⁺ T cells in vivo by a moderate static magnetic field

The above findings suggested that a static magnetic field enhances killing of CD8⁺ T cell by promoting the secretion of granzymes and cytokines. Since CD8+ T cells have been shown to play an important role in antitumor function by releasing granzymes and cytokines, we next used a tumor model to verify the effect of a static magnetic field on the antitumor function of CD8⁺ T cells *in vivo.* We performed studies using PyMT transgenic mice (spontaneous breast cancer) as a model. Button magnets of 0.3 T and 0.6 T were embedded into resin fiber plates (Fig 1, C) and placed into the mouse cage, whereas controls were blank plates placed without the embedded magnet, and PyMT female littermates were treated with magnetic plates or blank plates beginning at day 21. It is worth noting that the surface magnetic field strength of this button magnet (1 cm in diameter) is unevenly distributed, with a much higher magnetic field strength at the center of the magnet than at the edge. Overall, the surface magnetic field of the mosaic 0.3 T magnet fiber plate averaged ~ 0.18 T and that of the mosaic 0.6 T magnet fiber plate averaged ~ 0.31 T.

First, we observed the effect of the two magnetic plate treatments with different intensities on the time to tumor onset in mice. The results showed that there was no significant difference in time to tumor onset between mice treated with 0.3 T magnetic plates and control mice (Fig. 12, A); whereas the 0.6 T magnetic plate treated group showed a significant lag in time to tumor onset compared with the control mice (Fig. 13, A). Subsequently, we examined the effect of the magnetic plate treatment on tumor growth, which showed no significant difference in tumor growth curves between mice treated with 0.3 T magnetic plates and controls (Fig. 12, B); whereas tumor growth was significantly retarded in the 0.6 T magnetic plate treated group compared with the control mice (Fig. 13, B). In addition, we performed HE staining of tumor tissue sections, which showed that most cells in the control mice were tumor cells and the 0.6 T magnetic plate treated mice had significantly more retarded tumor development than the control mice, with more normal mammary tissue (Fig. 13, C). These results indicated that treatment with 0.6 T magnetic plates significantly inhibited tumor growth.

Subsequently, we examined whether magnetic field treatment affected tumor growth by affecting the function of tumor infiltrating CD8⁺ T cells. First, we compared the proportion of subsets of tumor infiltrating T cells in 0.6 T magnetic plate treated (indicated by SMF in the figure) and control mice and showed that the proportions of CD4⁺ T, CD8⁺ T, as well as the CD8⁺/CD4⁺ T cells did not differ significantly between magnetic field treated and control mice (Fig. 13, D). Subsequently, we examined the activation of tumor infiltrating T cells and showed that the proportions of activated CD8⁺ T cells, including CD44^{+high,} CD69⁺, as well as CD25⁺CD8⁺ T cells, were not significantly different between magnetic field treated and control mice (Fig. 13, E). In addition, there was no obvious difference in the ratio of naive CD4⁺ T and CD8⁺ T cells (CD62L^{high} CD44^{low}) to effector T cells (CD62L^{low}CD44^{hlgh}) (Fig. 14, A, B). In addition, there were Treg cells in the tumor immune microenvironment with the function of inhibiting immune response as well as MDSCs (myeloid suppressive cells), which would also play a promoting role on the growth of tumor. We also analyzed these cells and showed that neither the proportion of Treg cells nor MDSC cells was significantly different in magnetic field treated mice and control mice (Fig. 14, C-F). Notably, magnetic field treated mice secreted higher levels of granzyme and cytokines, including granzyme B, IFNγ and TNFα, in tumor infiltrating CD8⁺ T cells than control mice (Fig. 13, F, G). However, the levels of cytokine secretion by tumor infiltrating CD4⁺ T cells were not significantly different (Fig. 14). As reviewed, a static magnetic field was able to enhance the antitumor capacity of CD8⁺ T cells *in vivo* by promoting the secretion of cellular granzymes and cytokines.

### 7. Transplantation of static magnetic field treated CTLs for tumor immunotherapy

Above experimental results showed that a static magnetic field could enhance the killing of CD8⁺ T cells both *in vitro* and *in vivo,* and finally we examined its antitumor effect by transplanting CTLs (killer T cells) treated with magnetic field to tumor inoculated mice. We used a melanoma mouse model subcutaneously inoculated with B16F10-OVA₂₅₇₋₂₀₄ cells by tail vein injection of mice with PBS, CTLs without magnetic field treatment (indicated by "control"in the figure), and magnetic field treated CTLs (indicated by "SMF"in the figure), respectively. CTLs cells were obtained by isolating CD8⁺ T cells from spleens of OT-I TCR transgenic mice and performing *in vitro* TCR stimulation (see Section 5). Our experimental results showed that mice injected with magnetic field treated CTLs exhibited greater antitumor ability manifested by much smaller tumors and longer survival of mice than mice injected with control CTLs (Fig 16, A, B). Next, we examined the antitumor capacity of transplation of static magnetic field treated CTLs with in combination with an anti-PD-1 antibody and showed that the combination resulted in more antitumor efficacy than monotherapy (Fig. 16, C, D). Furthermore, transplation of static magnetic field treated CTLs in combination with anti-PD-1 antibody exhibited stronger antitumor effects than transplation of control CTLs without static magnetic field treatment in combination with anti-PD-1 antibody (Fig. 16, C, D). These data demonstrated a significant antitumor effect of transplanting static magnetic field treated CTLs to tumor grafted mice and a stronger antitumor effect in combination with anti-PD-1 than monotherapy.

### V. Conclusion and discussion

(1) Our study reveals that a moderate static magnetic field promotes the secretion of granzymes and cytokines, as well as ATP and mitochondrial respiration levels, of CD8⁺ T cells by regulating the expression of mitochondrial respiratory chain genes. In knockdown of the mitochondrial respiratory chain genes Uqcrb and Ndufs6, it was able to suppress the magnetic field treatment induced rise in granzyme and cytokine secretion by CD8⁺ T cells, as well as the rise in the expression of ATP. This reveals an entirely new molecular mechanism by which magnetic fields act on CD8⁺ T cell function. Uqcrb is a subunit of mitochondrial complex III, and Ndufs6 is a subunit of mitochondrial complex I, wherein a mutation in either of them leads to mitochondrial malfunction. In addition, mitochondrial respiration has been reported to be important for the secretion of cytokines IFNγ and IL-2 by CD8⁺ T cells. In the absence of MCJ, a negative regulator of mitochondrial respiration, CD8⁺ T cells develop elevated levels of ATP and mitochondrial respiration, as well as elevated secretion of the cytokines IFNγ and IL-2. This illustrates that the elevated cytokine secretion by CD8⁺ T cells due to magnetic field treatment detected in our experiments may be a consequence of elevated ATP as well as mitochondrial respiration.
(2) Our study also suggests that the magnetic receptor candidate genes *Iscal* and *Cryl*/*Cry2* are involved in the regulation of mitochondrial respiratory chain genes Uqcrb and/or *Ndufs6.* Knockdown of *Iscal* or *Cryl*/*Cry2* results in downregulation of *Uqcrb* and/or *Ndufs6* expression levels, may inhibit upregulation of granzyme and cytokine secretion by CD8⁺ T cells in response to magnetic field treatment, and upregulation of ATP expression. Although *Iscal* and *Cryl*/*Cry2* have been identified as magnetic receptor candidates, the downstream signaling pathways and target molecules they act on are not clear. Our study establishes a new link between magnetic receptor candidate genes and mitochondrial respiratory chain genes, which provides new clues for understanding how cells receive magnetic signals and then translate into biological signals to elicit biological effects. Furthermore, our study establishes a novel link between magnetic field, mitochondrial ATP synthesis, and CD8⁺ T cell killing, revealing not only a novel immunophysiological effect but also a novel mechanism of CD8⁺ T cell killing by a magnetic field.
(3) We confirmed *in vivo* that a moderate static magnetic field enhances the antitumor effect of CD8⁺ T cells and that reinfusion of magnetic field treated CTLs cells *in vivo* has a significant antitumor effect. This developed a new physical approach based on magnetic field sensing to enhance the antitumor effects of T cells. In contrast to existing T cell (CAR-T) therapies for chimeric antigens, which do not require the transfer of DNA fragments into target cells by means of gene editing, this approach is a noninvasive means of manipulating cellular function.
(4) In this study, we examined *in vitro* the cellular effects of a moderate static magnetic field on CD4⁺ T and CD8⁺ T cells at different magnetic field strengths for different TCR stimulation periods. We found that a moderate static magnetic field enhanced granzyme and cytokine secretion by CD8⁺ T cells in TCR stimulation for 72 h, but there was no significant effect in TCR stimulation for 24 h and 48 h. However, the cytokine secretion of CD4⁺ T cells by the moderate static magnetic field did not change significantly at any of these time points. Furthermore, our *in vivo* experiments confirmed that a moderate static magnetic field (0.6 T magnetic plate treatment) promoted the secretion of granzymes and cytokines by tumor infiltrating CD8⁺ T cells but did not affect cytokine secretion by CD4⁺ T cells, whereas treatment with 0.3 T magnetic plates had no significant antitumor effect. These experimental results demonstrate that the biological effects of a moderate static magnetic field on CD4⁺ T cells and CD8⁺ T cells are different and that the magnetic field strength is also an important experimental parameter.

## Claims

1. An activated immune cell **characterized by** upregulated expression or activity of the mitochondrial respiratory chain related genes Uqcrb and/or Ndufs6 relative to wild-type immune cells.

2. The immune cell of claim 1, **characterized by** one or more features selected from the group consisting of:
the immune cell is selected from the group consisting of lymphocytes, dendritic cells, monocytes/macrophages, granulocytes, and mast cells,
the immune cell has an increased expression or secretion of perforin, granzymes, and/or cytokines,
the immune cell has an increased level of ATP, and
the immune cell has an enhanced cell killing capacity.

3. The immune cell of claim 1 or 2, the cell is treated with a magnetic field, preferably, the magnetic field is a static magnetic field with a strength of 1 mT-1.0 T, and/or
the treatment is subjecting the cell to the magnetic field for at least 48 h.

4. A pharmaceutical composition comprising an immune cell of any one of claims 1-3 and a pharmaceutically acceptable carrier.

5. A method of upregulating the expression or activity of mitochondrial respiratory chain related genes of a cell, or increasing the ATP level of a cell, comprises treating the cell with a magnetic field, preferably, the magnetic field is a static magnetic field.

6. The method of claim 5, **characterized in that** the method has one or more features selected from the group consisting of:
the magnetic field is a static magnetic field with a strength of 1 mT-1.0 T,
the cell is an immune cell, preferably selected from the group consisting of lymphocytes, dendritic cells, monocytes/macrophages, granulocytes, and mast cells,
the mitochondrial respiratory chain related genes are *Uqcrb* and/or *Ndufs6*,
the treatment comprises subjecting the cell to the magnetic field for at least 48 h.

7. Use of a magnetic field, an agent that upregulates the expression or activity of mitochondrial respiratory chain related genes, in the manufacture of an agent containing an immune cell or an agent that increases the killing activity of the immune cell, **characterized by**,
the magnetic field is a static magnetic field,
the agent that upregulates the expression or activity of mitochondrial respiratory chain related genes is a primer that recognizes mitochondrial respiratory chain related genes or a vector containing the coding sequences of the mitochondrial respiratory chain related genes, preferably, the mitochondrial respiratory chain related genes are *Uqcrb* and/or *Ndufs6.*

8. The use of claim 7, **characterized in that** the use has one or more features selected from the group consisting of:
the magnetic field is a static magnetic field with a strength of 1 mT-1.0 T,
the immune cell is subject to the magnetic field for at least 48 h,
the immune cell has an elevated cell killing activity,
the immune cell has increased expression or secretion of perforin, granzymes, and/or cytokines,
the immune cell is selected from the group consisting of lymphocytes, dendritic cells, monocytes/macrophages, granulocytes, and mast cells,
the granzyme is granzyme A and/or B,
the cytokines comprise one or more selected from the group consisting of interleukin (IL), colony stimulating factor (CSF), interferon (IFN), tumor necrosis factor (TNF), transforming growth factor-β family (TGF-β), growth factors (GFS), and chemokine family,
preferably, the sequence of the primer that recognizes the mitochondrial respiratory chain related genes comprises one or more sequences selected from SEQ ID Nos: 9, 10, 15, and 16.

9. Use of a magnetic field, an agent which upregulates the expression or activity of mitochondrial respiratory chain related genes, or an immune cell of any one of claims 1-3 in the manufacture of a medicament for the treatment of cancer, **characterized by**,
the magnetic field is static magnetic field, preferably with a strength of 1 mT-1.0 T,
the agent that upregulates the expression or activity of the mitochondrial respiratory chain related genes is a primer that recognizes the mitochondrial respiratory chain related genes or a vector containing the coding sequences of the mitochondrial respiratory chain related genes, preferably, *Uqcrb* and/or *Ndufs6.*

10. Use of a magnetic part in the manufacture of an agent containing an immune cell, or in the manufacture of a device for increasing the ATP level of cells *in vivo* or *in vitro,* promoting mitochondrial respiration of cells *in vivo* or *in vitro,* enhancing immune cell killing activity *in vivo* or *in vitro,* or in the manufacture of a device for the treatment of cancer, **characterized in that** the magnetic part is able to form a static magnetic field with a strength of 1 mT-1.0 T, preferably, the magnetic part has one or more features selected from the group consisting of:
the magnetic part comprises a body loaded with a magnet,
the magnet is a button magnet of 0.3 T or 0.6 T,
the body is a resin fiber plate,
the body is 0.1-5.0 cm thick, preferably 1.5 cm thick,
the magnet is 0.5-5 cm in diameter, preferably 1 cm in diameter,
the distance of each two magnets is 0.5-5 cm, preferably 2.5 cm,
the magnet is embedded within the body,
the magnetic part is positioned anywhere in the device, preferably at the bottom,
the device is a mammalian cage containing the magnetic part.
